# FASCICULE DE BREVET EUROPEEN

(11) **EP 0 781 344 B1**
(45) Date de publication et mention de la délivrance du brevet: **23.11.2005**
(21) Numéro de dépôt: 95930578.0
(22) Date de dépôt: 13.09.1995
(51) Int. Cl.: C12N 15/86, A61K 48/00, A61K 9/00, C12N 5/10

(54) **NOUVEL IMPLANT ET NOUVEAU VECTEUR POUR LE TRAITEMENT DES MALADIES ACQUISES**
IMPLANTAT UND VEKTOR ZUR BEHANDLUNG VON ERWORBENEN KRANKHEITEN
NOVEL IMPLANT AND NOVEL VECTOR FOR THE TREATMENT OF ACQUIRED DISEASES

(30) Priorité: 13.09.1994 FR 9410911
(43) Date de publication de la demande: 02.07.1997
(73) Titulaire: TRANSGENE S.A., 67000 Strasbourg (FR)
(72) Inventeur: LEROY, Pierre, F-67000 Strasbourg (FR); MEHTALI, Majid, 67115 Plobsheim (FR)
(74) Mandataire: Warcoin, Jacques
(86) Numéro de dépôt international: PCT/FR1995/001171
(87) Numéro de publication internationale: WO 1996/008574

(56) Documents cités:
- WO-A-90/01550
- WO-A-92/15676
- WO-A-93/03143
- WO-A-94/06920
- WO-A-94/10323
- WO-A-94/19017
- FR-A- 2 706 486
- US-A- 5 219 740
- HUMAN GENE THERAPY, vol. 5, no. 5, NEW YORK, NY, TATS UNIS, pages 595-601, S-Y. CHEN ET AL. 'Intracellular antibodies as a new class of therapeutic molecules for gene therapy.'
- PROCEEDINGS OF THE NATIONAL ACADEMY OF SCIENCES OF THE USA, vol. 91, no. 10, 10 Mai 1994 WASHINGTON, DC, TATS UNIS, pages 4318-4322, D. MORITZ ET AL. 'Cytotoxic T lymphocytes with a grafted recognition specificity for ERBB2-expressing tumor cells.'
- PROCEEDINGS OF THE NATIONAL ACADEMY OF SCIENCES, vol. 91, no. 8, 12 Avril 1994 WASHINGTON, DC, TATS-UNIS, pages 3348-3352, A. CONLEY ET AL. 'Neutralization of divergent human immunodeficiency virus type 1 variants and primary isolates by IAM-41-2F5, an anti-gp41 human monoclonal antibody.'
- JOURNAL OF CELLULAR BIOCHEMISTRY, SUPPLEMENT, no. 17 part E, 29 - 25 Avril 1993 NEW YORK, NY, TATS-UNIS, page 251 M. ROSENFELD ET AL. 'E1-,E3- replication deficient recombinant adenovirus vector containing the human cystic fibrosis transmembrane conductance regulator (CFTR) cDNA does not replicate in human respiratory epithelial cells.'

## Description

La présente invention concerne un nouveau type d'implant et son utilisation pour le traitement et la prévention du cancer ou du SIDA. Plus particulièrement, elle a pour objet un implant comprenant des cellules génétiquement modifiées capables d'exprimer et de sécréter des anticorps spécifiques reconnaissant les cellules cancéreuses ou les cellules infectées afin d'inhiber au moins partiellement leur division ou propagation ainsi que la production de particules virales dans les cellules infectées. La présente invention concerne également un vecteur adénoviral capable de diriger l'expression d'une protéine d'intérêt multimérique ainsi que d'un anticorps ou un de ses dérivés.

La possibilité de traitements de maladies humaines par thérapie génique est passée en quelques années du stade des considérations théoriques à celui des applications cliniques. Le premier protocole appliqué à l'homme a ainsi été initié aux Etats-Unis en septembre 1990 sur un patient génétiquement immunodéficient en raison d'une mutation affectant le gène codant pour l'Adénine Désaminase (ADA). Le succès relatif de cette première expérimentation a encouragé le développement de nouveaux protocoles de thérapie génétique pour diverses maladies génétiques ou acquises. Ceux qui sont en expérimentation à l'heure actuelle, consiste pour la plupart à transférer *ex vivo* le gène thérapeutique dans des cellules du patient, par exemples les cellules souches de la lignée hématopoïétique, puis de réinfuser au malade ces cellules corrigées. Il s'agit donc d'une technologie lourde, non réversible et qui présente le risque de réimplanter des cellules transformées.

Initiée plus récemment, la technologie des néo-organes permet de pallier les inconvénients majeurs des protocoles classiques de thérapie génique. Elle est basée sur la réimplantation chez le patient d'une structure artificielle, que l'on peut appeler "implant" et comprenant des cellules vivantes, véritables "micro-usines" permettant de délivrer *in vivo* et de manière continue la molécule thérapeutique d'intérêt.

Plus précisément, cette structure artificielle est constituée de cellules vivantes préalablement transduites par un vecteur viral portant le gène thérapeutique, qui sont incluses dans un gel de collagène enrobant une ossature de fibres synthétiques d'un matériau biocompatible (PTFE, polytétrafluoroéthylène ou Gore-TexTM). Ce gel contient également un facteur de croissance angiogénique (bFGF, *basic Fibroblast Growth Factor*). Après sa réimplantation chez l'animal, le néo-organe se vascularise généralement en quelques jours grâce aux propriétés angiogéniques et trophiques du bFGF. Il évolue alors vers une structure autonome, munie d'un tissu conjonctif, parfois innervée, et reliée à la circulation dans laquelle sont déversées les molécules thérapeutiques.

La possibilité d'utiliser les néo-organes pour la thérapie génique a déjà été évoquée dans plusieurs articles scientifiques ainsi que dans la demande internationale WO 92/15676. Cependant, la technologie divulguée dans les documents de l'art antérieur ne s'adresse qu'au traitement des maladies génétiques monogéniques résultant de l'expression défectueuse et innée d'un seul gène, et n'a, par conséquent, été mise en oeuvre que pour la sécrétion de molécules thérapeutiques monomériques comme le facteur IX, 1'α₁-antitrypsine, l'ADA, l'érytropoïétine (EPO) et la β-glucuronidase. Jusqu'à présent, cette technologie n'a pas été adaptée à la sécrétion de molécules thérapeutiques plus complexes comme les anticorps.

On a maintenant trouvé qu'un implant de fibroblastes génétiquement modifiés par un vecteur rétroviral pour l'expression des chaînes lourdes et légères d'un anticorps anti-VIH, une fois réimplanté dans une souris, est capable de sécréter de façon continue dans la circulation sanguine une quantité importante d'anticorps fonctionnels reconnaissant les cellules infectées portant à leur surface l'antigène contre lequel il est dirigé. La présente invention repose sur le fait qu'un fibroblaste est capable de produire des quantités à peu près stoechiométriques de chaînes lourdes et légères d'un anticorps susceptible ensuite de s'associer en tétramère pour former une molécule fonctionnelle. Elle offre la possibilité de traiter par immunothérapie les maladies acquises et notamment le SIDA et le cancer, deux maladies dont la complexité, la gravité ainsi que l'absence de traitements réellement satisfaisants, justifient le développement de technologies nouvelles, comme celle faisant l'objet de la présente invention.

La présente invention fournit également des vecteurs adénoviraux capables de diriger l'expression de molécules d'intérêt multimériques ainsi que d'anticorps et leurs dérivés. Ils peuvent être utilisés pour produire une immunotoxine dirigée contre le virus VIH et induire la destruction sélective de cellules infectées.

C'est pourquoi la présente invention a pour objet :
un implant de cellules génétiquement modifiées comprenant une séquence nucléotidique exogène codant pour tout ou partie d'un anticorps dirigé contre un antigène tumoral ou un epitope spécifique d'un virus ladite séquence nucléotidique exogène étant placée sous le contrôle des éléments nécessaires à son expression et à la sécrétion dudit anticorps, lesdites cellules étant fixées à une matrice extracellulaire et ledit anticorps sécrété par ledit implant étant capable de lier spécifiquement ledit antigène ou ledit épitope contre lequel il est dirigé.

Au sens de la présente invention, un implant, désigne tout ensemble de cellules vivantes génétiquement modifiées; telles que définies ci-après et destinées à être implantées dans le corps humain ou animal. On préfère tout particulièrement le cas où les cellules sont fixées à une matrice extra cellulaire, le tout formant une structure biocompatible et vascularisable. La matrice est de préférence composée de collagène. Mais d'autres matériaux peuvent être utilisés dans le cadre de la présente invention dans la mesure où ils sont biocompatibles. Elle comprend notamment (1) un support biocompatible comme des fibres synthétiques PTFE (polytétrafluoroéthylène ou Gore-Tex) revêtues d'un film collagénique afin de permettre l'adhésion cellulaire (2), un gel de collagène dans lequel les cellules au sein de l'implant sont incluses et (3) un agent angiogénique favorisant la vascularisation dans l'hôte. Le terme implant est un terme générique qui inclut notamment les néo-organes et les organoïdes.

Par ailleurs, il peut également s'agir d'implants encapsulés c'est à dire inclus dans une membrane de porosité contrôlée empêchant notamment le passage des cellules (cellules de l'implant et cellules du système immunitaire de l'hôte) mais permettant la diffusion de la molécule thérapeutique, des nutriments et des déchets.

Le terme "cellule génétiquement modifiée" fait référence à une cellule ayant incorporé du matériel génétique exogène. Ce dernier peut être inséré dans le génome de la cellule ou être présent sous forme d'épisome soit dans le cytoplasme ou dans le noyau cellulaire. La technologie pour introduire un matériel génétique exogène dans une cellule est conventionnelle et à la portée de l'homme de l'art. A cet égard, de nombreux vecteurs ont été développés et sont largement décrits dans les ouvrages de base de biologie moléculaire accessibles à l'homme de l'art.

Les cellules génétiquement modifiées en usage dans le cadre de la présente invention, comprennent notamment une séquence nucléotidique exogène. Cette dernière peut être une séquence naturelle (déjà présente dans le génome de la cellule hôte) ou hétérologue mais elle aura été introduite dans les cellules hôtes par les techniques de génie génétique (donc d'une manière exogène). On préfère tout particulièrement une séquence codant pour un produit qui n'est normalement pas exprimé dans celle-ci ou, si il l'est, à des concentrations physiologiques faibles. Conformément aux buts poursuivis par la présente invention, la séquence nucléotidique exogène code pour tout ou partie d'un anticorps. Un anticorps est une protéine (immunoglobuline) normalement produite par les lymphocytes B et qui reconnaît un antigène étranger particulier et déclenche la réponse immunitaire. Un anticorps natif est un tétramère composé de quatre chaînes protéiques : deux chaînes légères (L) et deux chaînes lourdes (H pour heavy en anglais) associées entre elles par des ponts disulfure. La chaîne légère est constituée d'une région variable (V_{L}) en position N-terminale et d'une région constante (C_{L}) en position C-terminale alors que la chaîne lourde comprend du N vers le C-terminal une région variable (V_{H}) suivie de trois régions constantes (C_{H1}, C_{H2} et C_{H3}). Les régions correspondantes des chaînes légères et lourdes s'associent pour former des domaines distincts. Le domaine variable, formé par l'association des régions variables des chaînes légères et lourdes d'une immunoglobuline, est responsable de la reconnaissance de l'antigène correspondant. Les domaines constants exercent des fonctions effectrices impliquées dans le déroulement de la réponse immunitaire.

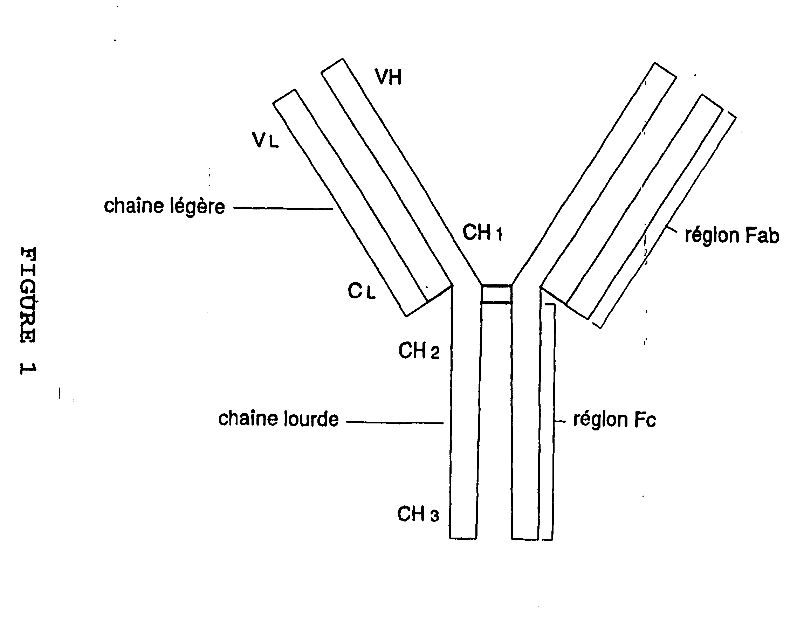

Aux fins de la présente invention, les deux chaînes lourdes et légères peuvent être identiques (anticorps natifs). Dans ce contexte, on emploie une séquence nucléotidique exogène codant pour une chaîne lourde et une chaîne légère qui s'associeront en tétramère après leur synthèse. Mais on peut également mettre en oeuvre une séquence codant pour une partie seulement d'un anticorps de manière à produire, de préférence, un fragment Fab (ab pour antigen binding en anglais) ou F(ab')₂, Fc (c pour cristallisable) ou encore scFv (sc pour simple chaîne et v pour variable). De tels fragments sont décrits en détail dans les ouvrages d'immunologie comme Immunology (third edition, 1993, Roitt, Brostoff et Male, ed Gambli, Mosby) et sont représentés schématiquement dans la Figure 1. En ce qui concerne plus spécifiquement le fragment scFv, celui-ci peut être obtenu d'une séquence codant pour une région V_{L} suivie d'une région V_{H} avec éventuellement un espaceur (de 1 à 10 résidus acides aminés neutres et peu encombrants) entre les séquences V_{L} et V_{H}.

On peut également générer un anticorps chimérique (ou hybride) provenant de la fusion de séquences d'origines diverses (espèces ou types d'anticorps). En particulier, on peut inclure ou échanger des régions constantes issues d'anticorps d'isotopes différents afin de conférer à l'anticorps chimère des propriétés nouvelles, par exemple une amélioration de la réaction cytotoxique. Il peut également s'agir d'un anticorps humanisé combinant au moins une partie des régions variables d'un anticorps de souris et des régions constantes d'un anticorps humain. On peut également fusionner une ou plusieurs régions ou parties de régions variables et/ou constantes d'une origine quelconque, par exemple issues de chaînes légères et/ou lourdes sous forme d'une molécule simple chaîne.

Enfin, une autre approche consiste à produire un anticorps bispécifique comportant deux domaines variables, par exemple un domaine reconnaissant un antigène porté par une cellule tumorale ou infectée et l'autre une structure d'activation de la réponse immunitaire. Ceci permet d'augmenter l'activité des cellules tueuses au contact de la tumeur ou de la cellule infectée.

Il va sans dire qu'un anticorps en usage dans la présente invention peut présenter une séquence légèrement différente de la séquence native d'un anticorps. En pratique, le dénominateur commun pour qualifier un anticorps est sa fonction, c'est à dire sa capacité à se lier spécifiquement à l'antigène contre lequel il est dirigé. De nombreuses techniques figurant dans les ouvrages généraux d'immunologie, permettent de mettre en évidence une fonction anticorps, par exemple les techniques ELISA, Western ou de fluorescence. L'invention s'étend à un anticorps dont la séquence a un degré d'homologie avec la ou les séquence(s) natives(s) (dans le cas d'un anticorps chimérique) supérieur à 70%, de manière avantageuse supérieur à 80%, de manière préférée supérieur à 90% et, de manière tout à fait préférée, supérieur à 95%. Un tel analogue peut être obtenu par mutation, délétion, substitution et/ou addition d'un ou plusieurs nuctéotide(s) de la ou des séquence(s) correspondante(s).

Conformément aux buts poursuivis par la présente invention, on préfère mettre en oeuvre un anticorps dirigé contre un antigène tumoral ou un épitope spécifique d'un microorganisme infectieux et pathogène, notamment d'un virus et plus particulièrement du virus VIH et avantageusement un antigène fortement représenté à la surface de la cellule cible. Ce type d'anticorps est largement décrit dans la littérature. On peut citer notamment :
- l'anticorps monoclonal humain 2F5 (Buchacher et al., 1992, Vaccines, 92, 191-195) reconnaissant un épitope continu (ELDKWAS) et très conservé de la glycoprotéine transmembranaire gp41 de la molécule d'enveloppe du VIH-1,
- l'anticorps monoclonal murin 17-1-A (Sun et al., 1987, Proc. Natl. Acad. Sci. USA, 84, 214-218) reconnaissant la glycoprotéine GA733 présente à la surface des cellules du carcinome colorectal humain,
- un anticorps dirigé contre la protéine MUC-1, et
- un anticorps dirigé contre la protéine E6 ou E7 du virus HPV (Human Papilloma virus) notamment de type 16 ou 18.

Dans le cadre de la présente invention, les séquences nucléotidiques codant pour un anticorps en usage dans le cadre de la présente invention, peuvent être obtenues par toute technique conventionnelle en usage dans le domaine du génie génétique, comme le PCR (Polymérase Chain Reaction), le clonage et la synthèse chimique. A titre purement indicatif, les séquences codant pour les chaînes lourdes et légères d'un anticorps peuvent être clonées par PCR en utilisant des oligonucléotides dégénérés reconnaissant les séquences conservées trouvées aux extrémités 5' et 3' de la plupart des gènes d'immunoglobulines (Persson et al., 1991, Proc. Natl. Acad. Sci. USA, 88, 2432-2436; Burton et al., 1991, Proc. Natl. Acad. Sci. USA, 88, 10134-10137). Puis on vérifie la fonction anticorps du produit d'expression vis à vis d'un antigène spécifique comme indiqué précédemment.

Une autre approche, par ailleurs préférée, consiste à utiliser un anticorps modifié, par fusion à une substance toxique ou une protéine immunopotentiatrice. Ce mode de réalisation spécifique permet de détruire *in vivo* par une chimiothérapie locale (substance toxique) la cellule cible (cellule cancéreuse ou cellule infectée) qui porte à sa surface l'antigène spécifique contre lequel la partie anticorps est dirigée ou d'améliorer la réaction immunitaire à son égard (substance immunopotentiatrice). Dans le contexte de la substance toxique, il peut être avantageux de choisir des anticorps qui peuvent être endocytés par la cellule ciblé. Il va sans dire que les séquences correspondantes peuvent être obtenues par toute technique classique dans le domaine de l'art.

Le terme "substance toxique" fait référence à une molécule ayant une activité dégradative inhibant drastiquement la croissance cellulaire ou induisant la mort cellulaire. Il peut s'agir d'une molécule toxique par elle-même ou de manière indirecte, par exemple une protéine catalysant la synthèse d'une substance toxique. Ces molécules peuvent être issues de plantes, d'animaux ou de microorganismes. Bien entendu, la fonction toxique peut être remplie par une substance toxique native (telle que trouvée dans la nature) ou un analogue de celle-ci, lequel peut être obtenu classiquement par mutation, délétion, substitution et/ou addition d'un ou plusieurs nucléotide(s) de la séquence native. Parmi les substances toxiques préférées, on peut citer une ribonucléase, la ricine, la toxine diphtérique, la toxine cholérique, la thymidine kinase du virus simplex de l'herpès de type 1 (TK-HSV-1), la cytosine déaminase d'*Escherichia coli* ou d'une levure du genre *Saccharomyces* et l'exotoxine de *Pseudomonas*.

Pour illustrer une protéine immunopotentiatrice (ayant pour fonction d'améliorer la réaction immunitaire de l'organisme hôte vis à vis de la cellule cible), on peut citer la protéine CD4, récepteur de haute affinité pour le virus VIH-1 ou un récepteur Fc pour IgG (FcγR). Son couplage à un anticorps dirigé contre un antigène du virus VIH ou tumoral permettra, par conséquent, de générer une molécule hybride disposant d'un ligand reconnaissant une cellule tueuse et d'un ligand reconnaissant la cellule cible afin de promouvoir plus efficacement son élimination. Dans ce cadre, on peut mettre en oeuvre une molécule hybride provenant de la fusion entre un anticorps anti-VIH et le FcγR ou entre le domaine extracellulaire de la molécule CD4 et un anticorps anti-CD3. Mais ces exemples ne sont pas limitatifs et de telles protéines immunopotentiatrices sont connues de l'homme de l'art.

Avantageusement, la fonction toxique est remplie par une ribonucléase, laquelle peut être d'origine procaryote ou eucaryote. Parmi celles utilisables dans le cadre de la présente invention, on peut citer la colicine E6, la cloacine d*'Escherichia coli,* la nucléase de *Staphylococcus,* la bimase de *Bacillus intermedius* et la nucléase de *Bacillus amyloliquefaciens,* encore désignée sous le nom de barnase, dont la séquence est divulguée dans Hartley (1988, J. Mol. Biol., *202,* 913-915). Mais, on préfère tout particulièrement mettre en oeuvre l'angiogénine humaine (Saxena et al., 1991, J. Biol. Chem., *266,* 21208-21214 ; Saxena et al., 1992, J. Biol. Chem., 267, 21982-21986).

Selon une autre variante, la fonction toxique peut être exercée par la TK-HSV-1. Celle-ci présente une affinité supérieure par rapport à l'enzyme TK mammifère pour certains analogues de nucléosides comme l'acyclovir et le ganciclovir et elle les convertit en précurseurs de nucléotides toxiques pour la cellule. Par conséquent, leur incorporation dans l'ADN des cellules en état de replication permet de tuer spécifiquement les cellules en division, comme les cellules cancéreuses, par un effet toxique et/ou par un effet de proximité (effet "by stander" en anglais).

Selon un autre mode de réalisation de l'invention, on peut mettre en oeuvre un analogue atténué présentant encore une fonction toxique mais moindre par rapport à la substance toxique native. Tout mutant présentant une activité dégradative atténuée peut être utilisé dans le cadre de l'invention. Dans ce contexte, on peut mettre en oeuvre un mutant atténué d'une ribonucléase présentant une activité atténuée d'un facteur 10 à 10⁶ ou mieux 10 à 10⁵ et, de manière tout à fait préférée, 10² à 10⁴ par rapport à la ribonucléase native dont il dérive. Cette variante est fondée sur l'importante toxicité des ribonucléases à l'égard des ARNs cellulaires, ce qui rend difficile les étapes de construction moléculaire. A titre d'exemples, on peut citer les mutants atténués de la barnase K27A (Mossakowska et al., 1989, Biochemistry, 28, 3843-3850) et K27A, L89F (Natsoulis et Boeke, 1991, Nature, *352,* 1632-1635). L'activité nucléase peut être évaluée conformément à la méthode décrite par Shapiro et al. (1987, Proc. Natl. Acad. Sci. USA, *84*, 8783-8787). Bien entendu, il est possible de la mesurer également par d'autres techniques, comme celle indiquée dans l'exemple 2.

Une construction particulièrement préférée consiste à inclure la séquence nucléotidique codant pour ladite substance toxique ou immunopotentiatrice en 5' ou en 3' de la séquence nucléotidique codant pour tout ou partie d'un anticorps. On préfère notamment le cas où elle est introduite en aval de la séquence codant pour la chaîne lourde d'un anticorps, cette dernière étant délétée du codon stop de la traduction et la fusion se faisant dans le bon cadre de lecture. La fusion de deux séquences de manière opérationnelle constitue une technique classique de biologie moléculaire à la portée de l'homme de l'art. Par ailleurs, on peut inclure au niveau de la fusion une séquence de liaison capable d'être clivée au sein de la cellule cible pour libérer la toxine. Dans ce contexte, le terme "séquence nucléotidique exogène" fait référence à une séquence codant pour tout ou partie d'un anticorps éventuellement fusionné à ladite substance.

Bien entendu, ladite séquence nucléotidique exogène est placée sous le contrôle des éléments nécessaires à son expression. Par "éléments nécessaires", on entend l'ensemble des éléments nécessaires à sa transcription en ARN messager (ARNm) et à la traduction de ce dernier en protéine. Parmi les éléments nécessaires à la transcription, le promoteur revêt une importance particulière. D'une façon générale, on aura recours à un promoteur fonctionnel dans une cellule encaryote et notamment humaine. Il peut s'agir d'un promoteur constitutif ou d'un promoteur régulable et il peut être isolé d'un gène quelconque d'origine eucaryote ou virale. Par ailleurs, un promoteur en usage dans la présente invention, peut être modifié de manière à contenir des séquences régulatrices comme des séquences activatrices de type "enhancer". Alternativement, on peut employer un promoteur dérivé des gènes d'immunoglobulines lorsque l'on cherche à cibler une cellule hôte lymphocytaire. Néanmoins, on préférera avoir recours à un promoteur constitutif permettant une expression dans un grand nombre de types cellulaires et, notamment, un promoteur d'un gène de maintenance comme le promoteur du gène TK-HSV-1, le promoteur adénoviral EIA, MLP (pour Major Late promoter en anglais), le promoteur PGK (phosphoglycérate kinase) murin ou humain, le promoteur du gène β-actine de rat (ACT), le promoteur HPRT (Hypoxantyl Phosphoribosyl Transferase), le promoteur HMG (Hydroxymethyl - Glutaryl coenzyme-A), le promoteur RSV (Rous Sarcoma Virus), le promoteur précoce du virus SV40 (Simian Virus) ou encore le promoteur DHFR (Dihydrofolate Réductase). A titre indicatif, lorsque la séquence nucléotidique est incorporée dans un vecteur rétroviral, le LTR 5' peut être utilisé comme promoteur. Cependant, on préfère tout particulièrement avoir recours à un promoteur non rétroviral interne, tels que ceux spécifiés auparavant.

La séquence nucléotidique exogène peut en outre contenir d'autres éléments contribuant à son expression tant au niveau de la transcription que de la traduction, notamment une séquence intronique bordée par les signaux d'épissage adéquates, une séquence de localisation nucléaire, une séquence d'initiation de la traduction, les éléments de terminaison de la transcription (signal de polyadénylation), et/ou une séquence codant pour un signal de sécrétion. Cette dernière peut être homologue c'est à dire issue du gène codant pour l'anticorps en question ou hétérologue c'est à dire dérivée d'un gène quelconque codant pour un précurseur d'un produit d'expression sécrété. Le choix de tels éléments est large et à la portée de l'homme de l'art.

Aux fins de la présente invention, la séquence nucléotidique exogène munie des éléments nécessaires à son expression est introduite dans une cellule hôte pour donner une cellule génétiquement modifiée. Tous les protocoles permettant d'introduire un acide nucléique dans une cellule peuvent être employés, comme par exemple la précipitation au phosphate de calcium, la technique du DEAE dextran, l'injection directe de l'acide nucléique dans la cellule hôte, le bombardement de microparticules d'or couvertes d'acide nucléique ou encore l'utilisation de liposomes ou de lipides cationiques. Toutefois dans le cadre de la présente invention, la séquence nucléotidique exogène est de préférence insérée dans un vecteur d'expression. En particulier, il peut être de type plasmidique ou dérivé d'un virus animal et notamment d'un rétrovirus, d'un adénovirus, d'un virus associé à l'adénovirus ou d'un virus de l'herpès. Toutefois, on préfère employer un vecteur intégratif. Le choix d'un tel vecteur est large et les techniques de clonage dans le vecteur retenu sont à la portée de l'homme de l'art. De même, il connaît le procédé à mettre en oeuvre pour générer des particules virales infectieuses.

Un premier vecteur particulièrement adapté à la présente invention est un vecteur adénoviral (voir ci-après).

Selon une autre alternative également avantageuse, on met en oeuvre un vecteur rétroviral. Les nombreux vecteurs décrits dans la littérature peuvent être employés dans le cadre de la présente invention et notamment ceux dérivés du virus de la leucémie murine de Moloney (MoMuLV) ou de Friend (FrMuLV). De manière générale, un vecteur rétroviral en usage dans la présente invention est délété de tout ou partie des gènes viraux *gag, pol* et/ou e*nv* et comprend un LTR 5', une région d'encapsidation et un LTR 3'. La séquence nucléotidique exogène est insérée de préférence en aval de la région d'encapsidation. La propagation d'un tel vecteur nécessite l'emploi de lignées de complémentation décrites dans l'art antérieur, telles que les lignées CRE, GP+E-86, PG13, Psi Env-am-12, pA317 et psi-CRIP.

Selon un mode de réalisation préféré et s'agissant de produire un anticorps autre que simple chaîne (comprenant par exemple deux chaînes protéiques lourdes et légères), on préfère avoir recours à un vecteur dicistronique permettant la synthèse des deux produits de traduction à partir d'un ARNm unique. L'initiation de la traduction du deuxième produit de traduction est, de préférence, assurée par un site IRES (pour Internal Ribosome Entry Site en anglais c'est à dire un site interne d'entrée des ribosomes). A ce jour, un certain nombre de sites IRES ont été identifiés et on peut citer celui du virus de la polyomélite (Pelletier et al., 1988, Mol. Cell. Biol., *8*, 1103-1112), de l'EMCV (Encephalomyocarditis Virus) (Jang et al., J. Virol., 1988, 62, 2636-2643) ou ceux décrits dans la demande internationale WO93/03143. Mais d'autres sites IRES peuvent également être employés. Ce type de construction peut être adapté à tout vecteur en usage dans le cadre de l'invention.

Un des vecteurs préférés dans le cadre de la présente invention est un vecteur rétroviral qui comprend de 5' vers 3' :
(a) un LTR 5' dérivé d'un rétrovirus,
(b) une région d'encapsidation,
(c) une séquence nucléotidique exogène comprenant :
   - un promoteur interne,
   - une première séquence codant pour la chaîne lourde d'un anticorps,
   - un site d'initiation de l'entrée des ribosomes,
   - une deuxième séquence codant pour la chaîne légère d'un anticorps, et
(d) un LTR 3' dérivé d'un rétrovirus.

Un autre vecteur rétroviral préféré comprend une séquence nucléotidique exogène pourvue du promoteur PGK murin suivi d'une première séquence codant pour les domaines 1 et II extracellulaires de la molécule CD4 et d'une seconde séquence fusionnée en phase à la première et codant pour le segment γ3 de la chaîne lourde de l'anticorps 2F5 (sCD4-2F5) et, de manière optionnelle, d'une troisième séquence codant pour l'angiogénine humaine liée à la seconde d'une manière opérationnelle.

Il va sans dire que l'ordre des premières, deuxièmes et troisièmes séquences peut être inversé. Par ailleurs, comme indiqué précédemment, la séquence nucléotidique exogène peut comprendre une séquence codant pour une substance toxique ou immunopotentiatrice. Celle-ci sera préférentiellement insérée en aval de la première séquence codant pour la chaîne lourde d'un anticorps. Cependant, la présente invention n'est pas limitée à ce mode de réalisation spécifique.

Par ailleurs, un vecteur en usage dans le cadre de l'invention peut également contenir d'autres éléments, par exemple, un gène codant pour un marqueur de sélection permettant de sélectionner ou d'identifier les cellules hôtes transfectées. On peut citer le gène *néo* conférant une résistance à l'antibiotique G418, le gène *dhfr,* le gène CAT (chloramphénicol Acétyl Transférase), le gène puromycine acetyl-transférase (*pac* ou PURO) ou encore le gène *gpt* (xanthine guanine phosphoribosyl transférase).

Une cellule génétiquement modifiée est de préférence choisie de manière à être tolérée par le système immunitaire de l'organisme hôte dans lequel on envisage de greffer un implant selon l'invention. Dans ce contexte, on préfère tout particulièrement une cellule non-tumorale et transfectable. Il peut notamment s'agir de cellules autologues prélevées ou dérivées de cet organisme hôte mais aussi de cellules pouvant être tolérées suite à un traitement adéquat chimique ou génétique (on peut par exemple envisager de réprimer l'expression des antigènes de surface normalement reconnus par le système immunitaire de l'organisme hôte). On peut également employer une cellule syngénique ou une cellule allogénique du même haplotype que l'organisme hôte en ce qui concerne les antigènes de classe II du complexe majeur d'histocompatibilité.

De manière préférée, une cellule génétiquement modifiée résulte de l'introduction de la séquence nucléotidique exogène dans des fibroblastes autologues et, en particulier, des fibroblastes prélevés de la peau d'un organisme hôte. Mais d'autres types cellulaires peuvent être utilisés, comme les cellules endothéliales, les myoblastes, les lymphocytes et les hépatocytes. Bien que cela ne soit pas un mode de réalisation préféré, on peut aussi avoir recours à des cellules tumorales (éventuellement atténuées par radiothérapie) prélevées d'un organisme hôte présentant des tumeurs, pour modifier leur patrimoine génétique et les rendre aptes à inhiber ou ralentir la progression tumorale.

Avantageusement, un implant selon l'invention comprend de 10⁶ à 10¹², de préférence de 10⁷ à 10¹¹ et, de manière tout à fait préférée de 10⁸ à 10¹⁰ cellules génétiquement modifiées.

La présente invention concerne également une méthode de préparation d'un implant selon l'invention dans laquelle on met en présence les cellules génétiquement modifiées et une matrice extracellulaire. Différentes techniques peuvent être mises en oeuvre pour générer un implant selon l'invention. On préfère procéder de la façon suivante : les cellules génétiquement modifiées sont mises en contact d'une solution de collagène liquide, de préférence de type I, d'un support biocompatible constitué par exemple de fibres synthétiques de Gore-Tex revêtues de collagène et d'au moins un facteur de croissance angiogénique, par exemple le bFGF ou le VEGF (Vascular Endothelial Growth Factor). L'ensemble est placé à 37°C afin que la solution de collagène forme un gel avec un maillage dense qui englobe les cellules puis cultivé 4 à 5 jours *in vitro* afin de permettre aux cellules génétiquement modifiées de coloniser l'implant. Il est souhaitable d'effectuer la dernière étape de culture dans un milieu contenant au moins un facteur angiogénique ou une combinaison de deux ou plus. D'une manière générale, les techniques qui permettent de générer un implant et les conditions de culture sont connues de l'homme du métier.

Un implant selon l'invention est destiné à être greffé dans un organisme hôte, animal ou, de préférence, humain afin d'y produire un effet thérapeutique (curatif et/ou préventif). Greffé à un animal de laboratoire, il permettra notamment d'évaluer les protocoles thérapeutiques applicables à l'homme. Le site de réimplantation est, de préférence, la cavité péritonéale ou sous cutané, intra-rachidien ou encore intra-abdominal.

L'invention s'étend également à l'usage thérapeutique d'un implant selon l'invention pour la préparation d'une composition pharmaceutique destinée plus particulièrement au traitement et/ou à la prévention d'une maladie acquise comme le cancer ou une maladie infectieuse causée par un microorganisme pathogène (virus, parasite ou bactérie). Elle s'adresse notamment au traitement:
- du cancer de l'utérus induit par un papilloma virus contre lequel on mettra en oeuvre un implant comprenant des fibroblastes autologues dans lesquels on a introduit une séquence codant pour un anticorps anti E6 ou E7 de HPV (en particulier de type 16 ou 18),
- du cancer du sein en mettant en oeuvre un anticorps anti-MUCI,
- du SIDA en mettant en oeuvre un anticorps dirigé contre un épitope de la glycoprotéine d'enveloppe conservé dans de nombreux isolats,
- de l'hépatite en mettant en oeuvre un anticorps dirigé contre un épitope du virus de l'hépatite B ou C.

Bien entendu, ces anticorps peuvent être modifiés par fusion notamment à l'angiogénine, la barnase ou la TK-HSV-1.

L'invention est également relative à une méthode de traitement ou de prévention des maladies acquises selon laquelle on génère *in vitro* un implant selon l'invenüon et on le greffe à un patient ayant besoin d'un tel traitement. Les sites de réimplantation peuvent être variés comme mentionné précédemment. Une fois que l'effet thérapeutique désiré est obtenu, il suffit de retirer chirurgicalement l'implant du patient.

Naturellement, les modalités du protocole thérapeutique doivent être mises au point par le clinicien en fonction du patient et de la maladie à traiter. Ce protocole peut être sujet à de nombreuses variantes comme le nombre d'implants selon l'invention à greffer, le site d'implantation et le type d'anticorps sécrété ainsi que le niveau d'expression. A titre purement indicatif, on préfère un niveau d'expression dans le sérum du patient d'au moins 50 ng/ml d'anticorps fonctionnel, avantageusement d'au moins 100 ng/ml, de préférence d'au moins 200 ng/ml et, de manière tout à fait préférée, d'au moins 500 ng/ml. Un anticorps fonctionnel est un anticorps capable de reconnaître l'antigène contre lequel il est dirigé. La fonctionnalité peut être mise en évidence par exemple par ELISA ou FACS. D'autre part, lorsque l'on met en oeuvre un anticorps fusionné à la TK-HSV-1, il est souhaitable d'inclure dans le protocole thérapeutique l'administration d'acyclovir ou de ganciclovir afin que son effet toxique puisse s'exercer.

Par ailleurs, la présente invention concerne un vecteur adénoviral recombinant comprenant une séquence nucléotidique exogène codant pour tout ou partie d'un anticorps dirigé contre un antigène tumoral ou un épitope spécifique d'un microorganisme infectieux et pathogène et modifié par fusion à une substance toxique ou immunopotentiatrice, ladite séquence nucléotidique étant placée sous le contrôle des éléments nécessaires à son expression et ledit anticorps étant capable de lier spécifiquement ledit antigène ou ledit épitope contre lequel il est dirigé. Elle concerne également un vecteur adénoviral recombinant comprenant une séquence nucléotidique exogène codant pour tout ou partie d'une ou plusieurs protéine(s) capable(s) de former un multimère dans une cellule hôte et, de préférence, un dimère ou un tétramère. Aux fins de la présente invention, un vecteur adénoviral recombinant selon l'invention peut être utilisé seul pour lutter contre une infection induite par un organisme pathogène ou rétablissement/propagation d'une tumeur dans un organisme ou une cellule hôte et la séquence nucléotidique exogène code pour tout ou partie d'au moins un anticorps dirigé contre un antigène tumoral ou un épitope spécifique d'un microorganisme infectieux et pathogène, ledit anticorps étant capable de lier spécifiquement ledit antigène ou ledit épitope contre lequel il est dirigé.

Un vecteur adénoviral recombinant selon l'invention dérive, de préférence, d'un adénovirus humain de sérotype C et, plus particulièrement, de type 2, 5 ou 7. Cependant, on peut également avoir recours à d'autres adénovirus, notamment d'origine animale (canine, bovine, murine, aviaire, ovine, porcine ou simienne) ou à un hybride entre des espèces variées. On peut citer plus particulièrement les adénovirus canins CAV-1 ou CAV-2, aviaires DAV ou encore bovins Bad de type 3 (Zakharchuk et al., 1993, Arch. Virol., 128, 171-176 ; Spibey et Cavanagh, 1989, J. Gen. Virol., 70, 165-172 ; Jouvenne et al., 1987, Gene, *60*, 21-28; Mittal et al., 1995, J. Gen. Virol., 76, 93-102). La technologie générale concernant les adénovirus est divulguée dans Graham et Prevec (1991, Methods in Mol. Biol., Vol 7, Gene Transfer and Expression Protocols, Ed : Murray, The Human Press Inc., p109-118).

Un mode de réalisation avantageux de la présente invention consiste à mettre en oeuvre un vecteur défectif pour une ou plusieurs fonction(s) virale(s) essentielle(s) à la replication, du fait de la délétion ou la non-fonctionnalité d'un ou plusieurs gènes viraux codant pour ladite fonction. Un tel vecteur, incapable de replication autonome, sera propagé dans une cellule de complémentation capable de fournir *en trans* les protéines, précoces et/ou tardives, qu'il ne peut lui même produire et qui sont nécessaires à la constitution d'une particule virale infectieuse. Ce dernier terme désigne une particule virale ayant la capacité d'infecter une cellule hôte et d'y faire pénétrer le génome viral. A titre illustratif, pour propager un vecteur adénoviral défectif pour la fonction E1, on aura recours à une cellule de complémentation telle que la lignée 293 capable de fournir *en trans* l'ensemble des protéines codées par la région E1 (Graham et al., 1977, J. Gen. Virol. 36, 59-72). Bien entendu, un vecteur selon l'invention peut comporter des délétions supplémentaires, notamment dans la région E3 non essentielle afin d'accroître les capacités de clonage mais également dans les régions E2, E4, L1-L5 essentielles (voir la demande internationale WO94/28152). Les fonctions défectives peuvent être complémentées à l'aide d'une lignée cellulaire ou d'un virus auxiliaire.

Un vecteur adénoviral préféré selon l'invention est délété de la majorité des régions E1 et E3 et porte, à la place de la région E1, une cassette d'expression comprenant :
(a) un promoteur, l'intron du gène de la β-globine humaine (BGL), les séquences codant pour la chaîne légère du 2F5, le site IRES du virus EMCV et la chaîne lourde du 2F5 puis le site de polyadénylation du gène de la β-globine humaine, ou
(b) un promoteur, l'intron du gène de la β-globine humaine, les séquences codant pour la molécule sCD4-2F5 éventuellement fusionnée en C-terminal et dans le même cadre de lecture à l'angiogénine humaine.

Parmi les promoteurs envisageables dans le cadre de la présente invention, on peut citer le promoteur précoce adénoviral EIA, le promoteur tardif MLP (Major Late Promoter), le promoteur murin ou humain PGK (Phosphoglycérate kinase), le promoteur précoce du virus SV40, le promoteur du virus RSV (Rous Sarcoma virus), un promoteur actif spécifiquement dans les cellules tumorales et enfin un promoteur actif spécifiquement dans les cellules infectées.

L'invention a également trait à une particule adénovirale infectieuse ainsi qu'à une cellule hôte eucaryote comprenant un vecteur adénoviral recombinant selon l'invention. Ladite cellule hôte est avantageusement une cellule de mammifère et, de préférence, une cellule humaine et peut comprendre ledit vecteur sous forme intégrée dans le génome ou non intégrée (épisome). Il peut s'agir d'une cellule primaire ou tumorale d'une origine hématopoïétique (cellule souche totipotente, leucocyte, lymphocyte, monocyte ou macrophage...), musculaire, hépatique, épithéliale ou fibroblaste.

Une particule virale infectieuse selon l'invention peut être préparée selon toute technique conventionnelle dans le domaine de l'art (Graham et Prevect, 1991, *supra*)*,* par exemple, par co-transfection d'un vecteur et d'un fragment adénoviral dans une cellule appropriée ou encore par le moyen d'un virus auxiliaire fournissant *en trans* les fonctions virales non fonctionnelles. II est également envisageable de générer le vecteur viral *in vitro* dans *Escherichia coli (E. coli)* par ligation ou encore recombinaison homologue (voir par exemple la demande française 94 14470).

L'invention a également pour objet une composition pharmaceutique comprenant à titre d'agent thérapeutique ou prophylactique, un vecteur adénoviral, une particule virale infectieuse ou une cellule hôte eucaryote selon l'invention en association avec un support acceptable d'un point de vue pharmaceutique. La composition selon l'invention est en particulier, destinée au traitement préventif ou curatif de maladies acquises telles que les cancers, les maladies virales comme le SIDA, l'hépatite B ou C ou les infections virales récurrentes provoquées par le virus de l'herpès.

Une composition pharmaceutique selon l'invention peut être fabriquée de manière conventionnelle. En particulier, on associe une quantité thérapeutiquement efficace d'un agent thérapeutique ou prophylactique à un support tel qu'un diluant. Une composition selon l'invention peut être administrée par voie locale, systémique ou par aérosol. On préfera notamment l'administration intramusculaire, intratumorale, intrapulmonaire et, tout particulièrement, l'injection intraveineuse. L'administration peut avoir lieu en dose unique ou répétée une ou plusieurs fois après un certain délai d'intervalle. La voie d'administration et le dosage appropriés varient en fonction de divers paramètres, par exemple, de l'individu ou de la maladie à traiter ou encore du ou des gène(s) d'intérêt à transférer. En particulier, les particules virales selon l'invention peuvent être formulées sous forme de doses comprises entre 10⁴ et 10¹⁴ ufp (unités formant des plages), avantageusement 10⁵ et 10¹³ ufp et, de préférence, 10⁶ et 10¹¹ ufp. La formulation peut également inclure un adjuvant ou un excipient acceptable d'un point de vue pharmaceutique.

Enfin, la présente invention est relative à l'usage thérapeutique ou prophylactique d'un vecteur adénoviral, d'une particule virale infectieuse ou d'une cellule hôte eucaryote selon l'invention pour la préparation d'un médicament destiné au traitement du corps humain ou animal et, préférentiellement, par thérapie génique. Selon une première possibilité, le médicament peut être administré directement *in vivo* (par exemple par injection intraveineuse, dans une tumeur accessible, dans les poumons par aérosol...). On peut également adopter l'approche *ex vivo* qui consiste à prélever des cellules du patient (cellules souches de la moëlle osseuse, lymphocytes du sang périphérique, cellules musculaires...), à les infecter *in vitro* selon les techniques de l'art et à les réadminister au patient.

L'invention est également relative à une méthode de traitement ou de prévention des maladies acquises selon laquelle on administre une quantité thérapeutiquement efficace d'un vecteur adénoviral recombinant, d'une particule adénovirale infectieuse ou d'une cellule hôte selon l'invention à un patient ayant besoin d'un tel traitement.

L'invention est illustrée sans pour autant être limitée par les exemples suivants et par référence aux figures suivantes :
La Figure 1 est une représentation schématique de la structure d'un anticorps et des fragments F(ab) et Fc.
La Figure 2 est une représentation schématique du vecteur pTG4370 permettant l'expression de l'anticorps 2F5.
La Figure 3 est une représentation schématique du vecteur pTG6356 permettant l'expression de l'anticorps 17-1-A couplé à la barnase native.
La Figure 4 est une représentation schématique du vecteur pTG6357 permettant l'expression de l'anticorps 17-1-A couplé à la barnase atténuée K27A.
La Figure 5 est une représentation schématique du vecteur pTG6355 permettant l'expression de l'anticorps 17-1-A.
La Figure 6 est une représentation schématique de la structure de la protéine membranaire CD4.
La Figure 7 est une représentation du schéma de construction des séquences codant pour la molécule hybride sCD4-2F5.
La Figure 8 est une représentation schématique du vecteur retroviral pTG8338 permettant l'expression de la molecule hybride sCD4-2F5.
La Figure 9 est une représentation schématique du vecteur pTG8373 comportant les séquences codant pour la molécule de fusion sCD4-2F5-Angiogénine.
La Figure 10 est une représentation schématique du vecteur adénoviral pTG8357 permettant l'expression de la molecule hybride sCD4-2F5.
La Figure 11 est une représentation schématique du vecteur adénoviral pTG8376 permettant l'expression de la molécule de fusion sCD4-2F5-Angiogénine.

### EXEMPLES

Les constructions ci-après décrites sont réalisées selon les techniques générales de génie génétique et de clonage moléculaire détaillées dans Maniatis et al. (1989, Laboratory Manual, Cold Spring Harbor Laboratory Press, Cold Spring Harbor, NY) ou selon les recommandations du fabricant lorsqu'on utilise un kit commercial. En ce qui concerne la réparation des sites de restriction, le remplissage des extrémités 5' protubérantes peut être réalisé à l'aide du fragment Klenow de l'ADN polymérase d'*Escherichia coli (E. Coli*) et la destruction des extrémités 3' protubérantes en présence de l'ADN polymérase du phage T4 ou par traitement par la nucléase S1 suivi d'une réparation par la Klenow. Les techniques de PCR sont connues de l'homme de l'art et abondamment décrites dans PCR Protocols, a guide to methods and applications (Ed : Innis, Gelfand, Sninsky et White, Academic Press, Inc.).

Les étapes de clonage mettant en oeuvre des plasmides bactériens sont effectuées de préférence par passage dans la souche *E. coli* XL1-Blue (Stratagène), et celles concernant des vecteurs dérivés du phage M13 dans E. *coli* NM522. On réalise les mutagénèses par oligodésoxynucléotides synthétiques à l'aide d'un kit d'origine commerciale (par exemple Amersham, RPN1523) et selon les recommandations du fabricant.

### Exemple 1 : Préparation d'un implant secrétant l'anticorps 2F5 et destiné à une immunothérapie anti SIDA

### A. Construction d'un vecteur rétroviral dicistronique pour l'expression et la sécrétion de l'anticorps 2F5 anti VIH.

Le vecteur à la base des constructions est le pLXSP qui dérive de pLXSN (Miller et Rosman, 1989, BioTechniques, 7, 980-988). Ce dernier est un vecteur rétroviral qui comprend le LTR 5' du MoMuSV (Moloney Murine Sarcoma Virus), une région d'encapsidation rétrovirale, des sites multiples de restriction, le gène *néo* de résistance à la néomycine sous le contrôle du promoteur SV40 et le LTR 3' du MoMuLV. Le vecteur pLXSP est obtenu après, d'une part, remplacement du fragment *Nhe*I *- Kpn*I du LTR 3' de pLXSN par un fragment analogue provenant du LTR 3' MPSV (Myélo Proliférative Sarcoma Virus) isolé du vecteur pMPSV.H-2K.IL-2R (Takeda et al., 1988, Growth Factors, *1*, 59-66) et, d'autre part, introduction du gène de résistance puromycine en remplacement du gène *néo.* Le gène puromycine est obtenu de pBabe Puro décrit dans Morgenstern et Land (1990, Nucleic Acids Res., *18*, 3587-3596).

Le vecteur pLXSP est digéré par *Eco*RI et *Hpa*I et on y introduit un fragment *Eco*RI*-Pst*I (après réparation du site PstI) isolé de pKJ-1 (Adra et al., 1987, Gene, 60, 65-74). Ce fragment porte le promoteur du gène PGK de souris. Après ligation, on obtient le vecteur pTG2663. Celui-ci est soumis à une digestion par les enzymes *Cla*I et *Bam*HI afin d'éliminer la cassette d'expression du gène puromycine. Après traitement à la Klenow et ligation, on obtient le vecteur pTG2673 dans lequel le site *Bam*HI est reconstitué.

Le vecteur pTG2676 est obtenu par clonage du fragment *Hind*III-*Eco*RI comprenant l'ADNc codant pour la chaîne légère (LC) de l'anticorps monoclonal 2F5 dans le plasmide Bluescript SK+ (Stratagène). Il peut-être cloné par PCR d'une banque d'ADNc issue d'ARNm de l'hybridome 2F5 (Buchacher et al., 1994, AIDS Research and Human Retroviruses, *10*, 359-369 ; Katinger, 1992, Septième Colloque des Cent Gardes, 299-303) en utilisant des amorces adéquates complémentaires aux séquences entourant le codon initiateur de la traduction et le codon stop, telles que les amorces OTG5168 et OTG5169 (SEQ ID NO: 1 et 2). On isole du pTG2676 un fragment *Xho*I*-Bam*HI portant l'ADNc LC 2F5 lequel est inséré dans le vecteur pTG2673 préalablement digéré par les mêmes enzymes, pour générer pTG4336.

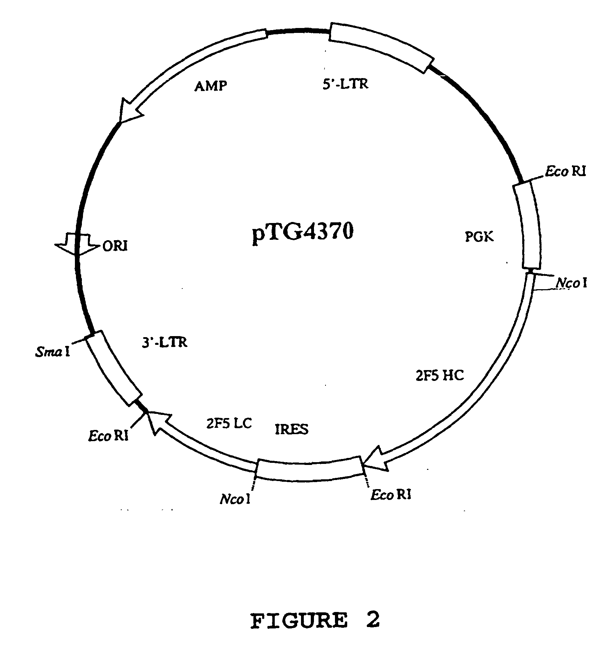

Ce dernier est linéarisé par l'enzyme *Nco*I et soumis à une ligation avec, d'une part, le fragment *Nco*I*-Eco*RI provenant du pTG2677 et portant l'ADNc codant pour la chaîne lourde (HC) de l'anticorps 2F5 et, d'autre part, le fragment *Eco*RI-*Nco*I purifié de pTG4369 et portant le site IRES de EMCV. Le vecteur pTG2677 est un pBluescript SK+ dans lequel on a introduit entre les sites *Hind*III et *Eco*RI l'ADNc HC 2F5 portant les mêmes extrémités. Ce dernier a été obtenu par PCR de la banque précédente à l'aide des amorces OTG5170 et OTG5171 (SEQ ID NO: 3 et 4). Quant au vecteur pTG4369, il provient du clonage également dans le pBluescript SK+, du fragment *Xha*I*-Cla*I correspondant à l'IRES EMCV (Jang et al., 1988, *supra*)*.* La triple ligation génère le vecteur pTG4370 (Figure 2). On peut, de manière optionnelle, réintroduire la cassette d'expression du gène puromycine dans la partie plasmidique de pTG4370 afin de faciliter les étapes de sélection. On obtient pTG6368.

On génère des particules virales infectieuses de la façon suivante :

La lignée de complémentation écotrope GP+E-86 (Markowitz et al., 1988, J. Virol., 62, 1120-1124) et les cellules cibles NIH3T3 (cellules fibroblastiques de souris) disponibles à l'ATCC, sont cultivées à 37°C en présence de 5% de CO₂ dans du milieu DMEM (Dulbecco's Modified Eagle's Medium) contenant 10% de sérum de veau foetal (SVF) (GibcoBRL), 1 mM Glutamine, 1 % d'acides aminés non essentiels et 40 µg/l de gentamycine (milieu DMEM complet). La veille de la transfection, les cellules GP+E-86 sont mises en culture à raison de 5 × 10⁵ cellules par boîte de 10cm. Le lendemain, 20 µg de plasmide pTG4370 linéarisé et 1 µg de vecteur de sélection (par exemple le vecteur pLXSP portant le gène de résistance à la puromycine) sont transfectés selon la méthode traditionnelle au phosphate de calcium. Le jour suivant (J+1) les cellules sont lavées selon les méthodes de l'art et placées dans un milieu neuf pendant 48 heures, avant d'être cultivées à partir de J+3 en milieu sélectif (5 µg/ml de puromycine).

Au terme d'environ 2 semaines de sélection, des clones cellulaires résistants à l'antibiotique sont visibles sur boîte. Ils sont isolés selon les techniques de l'art et les cellules resuspendues en milieu sélectif dans des plaques de culture à 96 puits. On sélectionne les clones meilleurs producteurs d'anticorps en mettant en oeuvre la méthode ELISA décrite ci-après et on titre les surnageants sur cellules cibles NIH3T3. Pour ce faire, la veille de l'infection, celles-ci sont ensemencées à 10⁵ cellules par puit. Les infections virales sont effectuées selon le protocole classique décrit dans la littérature. La méthode de titration est celle dite du point limite. La méthode ELISA permet de titrer la quantité d'anticorps fonctionnels 2F5 reconnaissant l'epitope cible (ELDKWAS). Celui-ci est synthétisé chimiquement.

Brièvement, une solution de peptide (2 mg/ml) est diluée 2000 fois dans un tampon (Na₂ CO₃ 15mM. NaHCO₃ 35mM, pH 9,6) et 100 µl sont déposés au fond de chaque puit d'une plaque de microtitration et incubés à 4°C pendant 16h. Après des lavages extensifs dans un tampon PBS, 0,05 % Tween-20, les puits sont saturés par 50 µl de BSA 1% dissoute dans un tampon PBS 1 h à 37°C. Après lavage, on ajoute 100 µl de l'échantillon à tester ou d'une solution d'étalonnage. La plaque est incubée 2h à température ambiante et lavée extensivement avec le tampon PBS-Tween 20. Puis on ajoute 100 µl d'un anticorps de chèvre anti IgG humaine conjugué à la péroxydase (concentration 0,8 mg/ml ; Jackson Immuno Research Laboratories Inc, PA) dilué 1000 fois dans un tampon PBS, 1 % BSA. Après 2 heures à température ambiante et lavages extensifs, l'activité enzymatique péroxydase est révélée par addition de 100 µl de la préparation suivante (Na₂HPO₄ 0,066 M, C₆H₈O₇ 0,035M pH5, 0,04 % d'orthophenylendiamine et 0,014 % de peroxyde d'hydrogène). La réaction est stoppée par 150 µl de H₂SO₄ 1M. L'absorbance est mesurée à 490 nm.

La solution d'étalonnage est obtenue soit d'une source commerciale (Virus Testing Systems, Houston) ou d'un surnageant d'hybridome concentré sur Centricon. La solution d'anticorps (1 µg/ml) est diluée de 2 en 2 dans du sérum de veau foetal. L'absorbance est mesurée pour chacune des dilutions et la courbe d'étalonnage est établie (ng d'anticorps en fonction de l'absorbance). On détermine ainsi les clones les plus producteurs.

### B. Préparation de l'implant

### 1/ Prélèvement et mise en culture de fibroblastes primaires.

Les biopsies de peau sont effectuées sur des souriceaux femelles BALB/C de 2 à 3 jours. Mais d'autres lignées de souris peuvent également convenir. Après une dissociation mécanique grossière, on place le prélèvement dans 30 ml de milieu DMEM complet en présence de 5000 unités de dispase (Collaborative Medical Products) et de 1 % de collagénase (Sigma). Après 2 heures à 37°C, on dilue le mélange et les cellules sont récoltées par centrifugation, lavées soigneusement avant d'être resuspendues dans du milieu RPMI 1640 (Gibco BRL). Après environ une semaine de culture, les fibroblastes primaires sont infectés par les surnageants de culture des clones producteurs sélectionnés comme à l'étape précédente (A) selon le protocole classique. On peut également réimplanter des cellules fibroblastiques NIH3T3 à titre de modèle. Elles sont cultivées comme indiquées précédemment et infectées de manière conventionnelle. La présence de l'anticorps 2F5 dans les surnageants de cellules NIH3T3 infectées par un des clones producteurs, a été suivie par test ELISA et a été évaluée à 500 ng/ml/24h soit une productivité de plus de 1 µg/10⁶ cellules /24h.

### 2/ Préparation d'un néo-organe

Les fibres de PTFE préalablement autoclavées (Gore Inc, AZ) sont tout d'abord mises en présence d'une solution de collagène de queue de rat (solution à 0,5 mg/ml dans de l'acide acétique 0,1 N) pendant 2 heures et sous vide. Elles sont ensuite étalées au fond d'un puit (plaque de 12 puits), stérilisées aux UV, réhydratées par du tampon PBS pendant une nuit avant d'être traitées 2 heures à température ambiante par des facteurs angiogéniques (10 ml de PBS contenant 2 µg de bFGF et 1 µg de VEGF pour environ 100 mg de fibres).

Parallèlement, les fibroblastes (primaires ou NIH3T3) infectés sont trypsinés brièvement. On resuspend 1,5 x 10⁷ cellules dans 0,2 ml de milieu puis on ajoute 2 ml du mélange suivant par puit: 200 µl de RPMI 10x, 24 µl de bicarbonate de sodium 7,5 %, 5 µl d'Hepès 1 M, 20 µl de Gentamycine, 20 µl de Glutamine, 2 µl de bFGF (10 ng/µl), 2 µl d'EGF (Epidermal Growth Factor) (10 ng/µl), 1,5 ml de collagène (2 mg/ml), 12 µl de NaOH (10 N) et 15 µl d'H₂O. Après 30 à 60 minutes d'incubation à 37°C. on observe une polymérisation du mélange. Celui-ci est cultivé à 37°C pendant environ 4 jours, en présence, pour la dernière nuit, de facteurs angiogéniques (bFGF et VEGF).

### C. Réimplantation de l'implant

Un ou deux implants sont introduits dans la cavité péritonéale soit de souris femelles BALH/c ou de souris Swiss nude. Un mois après leur implantation, on vérifie qu'ils sont amarrés au tissu adipeux de la cavité abdominale et sont vascularisés. Par ailleurs, des échantillons de sang sont prélevés régulièrement dans le mois suivant l'implantation et le dosage de l'anticorps 2F5 par ELISA (selon la technique décrite précédemment) fait apparaître des valeurs de l'ordre de 20 ng/ml de sérum dans les souris syngéniques BALB/c et dépassant 100 ng/ml de sérum dans les souris nude. Le niveau d'anticorps se maintient sur une période de plus de 6 à 7 semaines après l'implantation.

L'efficacité des anticorps 2F5 à inhiber l'infection virale VIH est évaluée sur des souris SCID (Severe Combined Immuno Deficiency). Il s'agit de souris immunodéficientes ne possédant ni cellules T, ni cellules B matures qui, par ailleurs, peuvent être humanisées par introduction de cellules ou de tissus humains. Ce traitement les rend infectables par le VIH (Namikawa et al., 1988, Science 242, 1684-1686)

Un à deux néo-organes sécretant l'anticorps 2F5 sont implantés dans la cavité abdominale de souris SCID humanisées par injection intraperitonéale de cellules lymphocytaires humaines CEM A3 (40x 10⁶ cellules). 3 à 5 semaines après la greffe, les souris sont éprouvées par le virus VIH (1000 TCID₅₀ de VIH1 isolat Bru par voie intraveineuse). Les cellules sont récupérées de l'animal 3 jours post infection et mises en cultures. Le surnageant cellulaire est prélevé à intervalles de temps régulier et l'activité reverse-transcriptase déterminée. On constate que les cellules humaines sont protégées contre l'infection par le VIH et que la protection est maintenue pendant toute la durée de l'expérience (50 jours). En effet, l'activité reverse transcriptase se situe en dessous du seuil de détection dans les souris greffées avec les implants secrétant l'anticorps 2F5 (comportement similaire au témoin non infecté). Ces données témoignent d'une inhibition de la replication du VIH dans les animaux produisant le 2F5 dans leur circulation sanguine.

### Exemple 2 : Préparation d'un implant pour une immunothérapie anti-cancer

### A. Construction du vecteur rétroviral dicistronique pour l'expression et la sécrétion de l'anticorps 17-1-A.

En premier lieu, le plasmide pBluescript SK+ est digéré par *NotI* puis soumis à un traitement par le grand fragment Klenow de l'ADN polymérase avant d'être religué sur lui-même. On génère le vecteur pTG6336 dans lequel le site *NotI* a été détruit. Parallèlement, l'ADNc codant pour la chaîne légère de l'anticorps 17-1-A est isolé par PCR d'une banque d'ADNc construite à partir d'ARNm isolé des cellules de l'hybridome 17-1-A (Sun et al., 1987, Proc. Natl. Acad. Sci. USA, *84*, 214-218 ; Herlyn et al., 1979, Proc. Natl. Acad. Sci. USA 76, 1438-1442). A titre indicatif, cet anticorps est dirigé contre un épitope de la glycoprotéine transmembranaire GA733-2 (Szala et al., 1990, Proc. Natl. Acad. Sci. USA, *87*, 3542-3546) présente à la surface des cellules du carcinome colorectal humain. Le PCR met en oeuvre les amorces OTG6114 et OTG6115 (SEQ ID NO : 5 et 6) conçues de manière à introduire des sites de restriction facilitant les étapes de clonage ultérieures, respectivement les sites *Eco*RI et *Nco*I en 5' et les sites *Bgl*II et *Xba*I en 3'. Après vérification sur gel d'agarose, le fragment PCR ainsi généré est digéré par *Eco*RI et *Xba*I puis cloné dans le pTG6336 entre ces mêmes sites. On génère le pTG6339.

Ce dernier est digéré par *Eco*RI et *Nco*I et ligué au fragment *Eco*RI*-Nco*I de pTG4369 portant le site IRES, pour donner pTG6343. On introduit dans ce dernier, l'ADNc codant pour la chaîne lourde de l'anticorps 17-1-A dépourvue du codon stop à la place duquel on insère un petit espaceur codant pour les résidus Gly-Gly-Gly-Gly-Ser, L'ADNc HC 17-1-A est obtenu par PCR à partir de la banque précédente d'ADNc et en mettant en oeuvre les oligonucléotides OTG6192 et OTG6194 (SEQ ID NO:7 et 8). L'insertion du fragment PCR digéré par *SaI*I*-Eco*RI permet de générer le pTG6346.

Ce dernier est linéarisé par *Not*I puis ligué à un fragment *Not*I portant la séquence codant pour la barnase pour donner pTG6347. Le gène codant pour la barnase est obtenu par PCR, à partir d'une préparation d'ADN génomique de *Bacillus amyloliquefaciens* et des amorces OTG5147 et OTG5148 (SEQ ID NO: 9 et 10). Les oligonucléotides ont été conçus de manière à introduire un site de restriction *Not*I en 5' du codon correspondant au premier acide aminé de la barnase mature et en 3' du codon stop. On vérifie que la séquence du fragment PCR ainsi généré est conforme à celle publiée dans Hartley (1988, J. Mol. Biol., *202,* 913-915).

Parallèlement le fragment *Not*I est inséré dans un vecteur de type M13, par exemple le vecteur M13TG130 (Kieny et al., 1983, Gene, *26*, 91-99) dans lequel on a, au préalable, introduit un site *Not*I au sein des sites de clonage par mutagénèse dirigée. La modification de sites de restriction par mutagénèse dirigée est une technique connue de l'homme du métier. Le vecteur ainsi obtenu est soumis à une mutagénèse dirigée à l'aide de l'oligonucléotide OTG5299 (SEQ ID NO: 11), afin de modifier le résidu lysine (Lys ou K) en position 27 de la barnase native par un résidu alanine (Ala ou A). Puis on isole le fragment *Not*I modifié, qui est introduit comme précédemment dans le vecteur pTG6346 pour donner pTG6348.

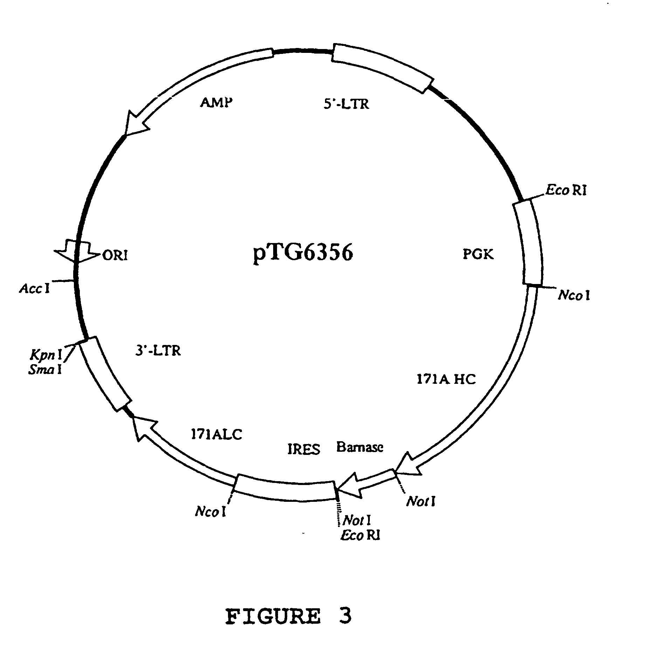

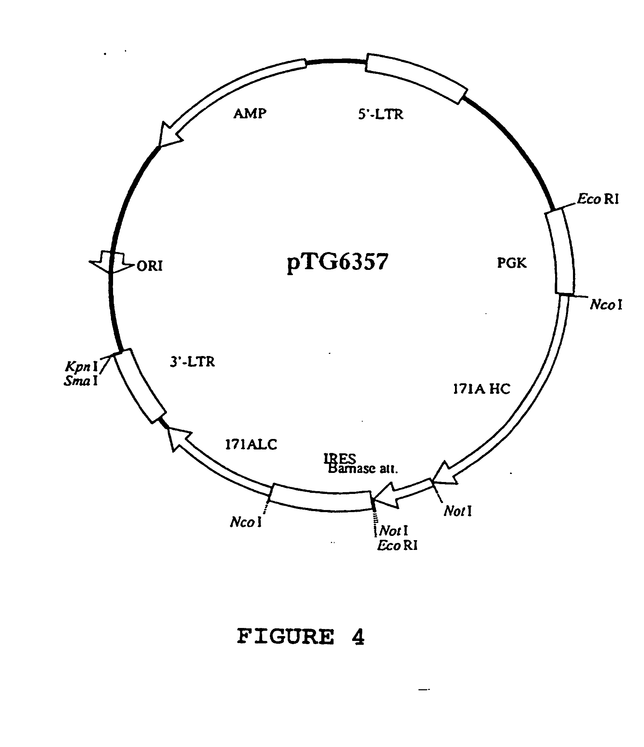

Le fragment *Sal*I *- Bgl*II isolé du vecteur pTG6347 ou pTG6348 est transféré dans le vecteur pTG2673 préalablement digéré par *Xho*I et *Bam*HI*.* On obtient respectivement les vecteurs pTG6356 et pTG6357 (Figures 3 et 4).

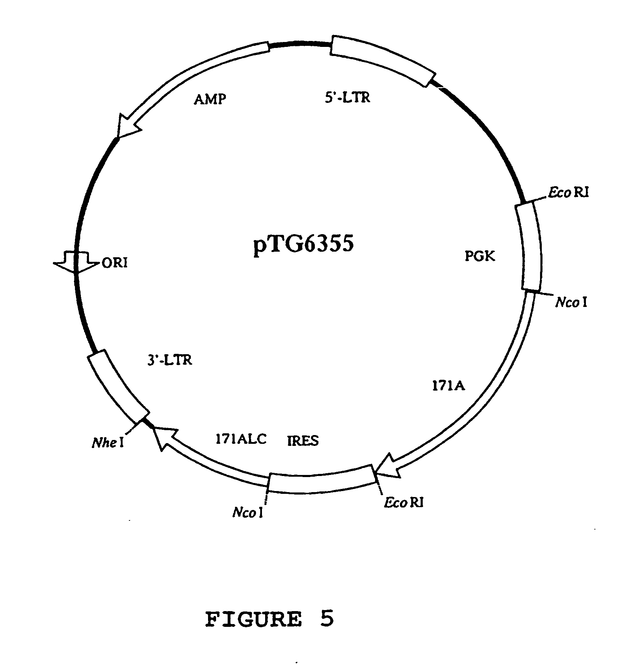

Par ailleurs, on construit un vecteur dicistronique contenant les séquences codant pour le HC 17-1-A, l'IRES EMCV suivi de LC 17-1-A. Le fragment HC pourvu d'un codon stop est obtenu par PCR du vecteur pTG6346 avec les oligonucléotides OTG6192 (SEQ ID NO: 7) et OTG6193 (SEQ ID NO: 12). Ce fragment PCR qui est muni de sites *Sal*I et *Eco*RI à ses extrémités 5' et 3' respectivement, est inséré dans le vecteur pTG6343 digéré par les mêmes enzymes pour donner le pTG6345. On clone le fragment *Sal*I*-Bgl*II de ce dernier entre les sites *Xho*I et *Bam*HI du pTG2673. On obtient le vecteur pTG6355 (Figure 5).

On génère des particules virales par transfection des cellules GP+E-86 avec les vecteurs pTG6355, pTG6356 et pTG6357 (cotransfection avec pLXSP) selon la technique décrite dans l'exemple 1, à la différence près que l'on teste les clones transfectés pour la production d'anticorps 17-1-A comme indiqué ci-après. 100 µl d'anticorps de chèvre anti immunoglobuline murine (Southern Biotechnology) préalablement dilués 100 fois dans un tampon (80 mM Na₂CO₃, 200 mM NaHCO₃, pH 9,6) sont répartis dans les puits d'une plaque de microtitration (Nunc) et incubés une nuit à 4°C. Les anticorps non adsorbés sont éliminés par lavages extensifs avec un tampon PB S 1X, 10 mM EDTA, 0,05 % Tween 20. Les puits sont saturés par addition de 200 µl d'une solution PBS 1X, 1 % BSA (Bovine Serum Albumine) 1h à 37°C. Après cette étape, la plaque est à nouveau lavée, puis la gamme de témoin (diluée dans du PBS 1X, 1% BSA) ou les surnageants de culture à tester sont déposés et incubés pendant 2 h à température ambiante sous agitation. Après plusieurs lavages, les plaques sont incubées avec 100 µl d'un anticorps de chèvre anti-Ig₂a, (isotype du 17-1-A), couplé à la biotine (Southern Biotechnology) dilué 5000 fois dans un tampon PBS 1X, 1 % BSA. Après 1 h d'incubation à température ambiante sous agitation, l'excès est éliminé par 8 lavages puis on distribue 100 µl d'une solution peroxidase-streptavidine (Amersham), diluée 1000 fois. Après une incubation de 45 min suivie de lavages extensifs; l'activité enzymatique est révélée par addition de 100 µl de la solution de substrat (pour une plaque : 12,5 ml de tampon 25 mM citrate, 50 mM Na₂HPO₄, pH 5; 1 pastille de 5 mg d'OPD (orthophénoldiamine, Sigma) ; 5 µl d'H₂O₂ 35 %). La réaction est stoppée par addition de 25 µl par puit de H₂SO₄ 3 M. L'absorbance est alors lue à 490 nm.

La quantité d'anticorps présente dans les surnageants de culture est établie en fonction d'une gamme d'étalonnage préparée comme suit : les cellules de l'hybridome 17-1-A sont injectées à des souris "nudes". Après formation des ascites, on prélève le liquide d'ascite à partir duquel on purifie l'anticorps 17-1-A par passage sur une colonne de protéine A sépharose. Il s'agit d'une technique conventionnelle à la portée de l'homme de métier. Une solution d'anticorps 17-1-A purifié est préparée à une concentration de 1 µg/ml puis diluée de 2 en 2 dans un tampon PBS. L'absorbance est mesurée pour chacune des dilutions et la courbe d'étalonnage est établie (ng d'anticorps en fonction de l'absorbance). Les clones les plus producteurs sécrètent, selon le vecteur transfecté, de 200 à 900 ng d'anticorps 17-1-A /10⁶ cellules/24 h.

### B. Préparation de l'implant

Les cellules NIH3T3 sont infectées par les clones les plus producteurs (issus de la transfection des cellules GP+E-86 par les vecteurs pTG6355, pTG6356 et pTG6357) et les surnageants de culture testés par ELISA pour la sécrétion d'anticorps 17-1-A. On détecte un taux d'anticorps variant de 100 à 1000 ng/10⁶ cellules /24 h selon les constructions.

Par ailleurs, on vérifie que l'anticorps produit reconnait l'antigène GA733 exprimé par les cellules SW948. Pour cela on met en oeuvre la technique de cytométrie de flux. Les cellules SW948 (ATCC CCL237) sont cultivées en milieu DMEM complet. Elles sont détachées par action de la trypsine, comptées puis 5x10⁵ cellules sont réparties dans des puits d'une plaque 96 puits. Cette plaque est centrifugée 1 min à 1000 rpm, sans freins, pour culotter les cellules. Le surnageant est éliminé puis la plaque est vortexée et les cellules resuspendues dans 100 µl de tampon FACS (PBS cationique 1X, 1 % BSA, 0,1 % γ globulines humaines, 5 mM EDTA). La plaque est à nouveau centrifugée et le surnageant éliminé. Les cellules sont alors resuspendues dans le surnageant de culture à tester ou dans une dilution de l'anticorps témoin dans du tampon FACS et incubées 1 h à 4°C. 4 lavages sont alors réalisés dans les conditions précédemment décrites puis les cellules sont resuspendues dans 100 µl d'un fragment F(ab')₂ de chèvre anti immunoglobuline de souris couplé à la fluoresceine (DTAF) (Jackson Immuno Research Laboratories) dilué 100 fois dans du tampon FACS. Une nouvelle incubation d'1 h à 4°C permet à l'anticorps de se fixer. L'excès est alors éliminé par 4 lavages et les cellules sont finalement resuspendues dans 300 µl de PBS cationique 1X avant d'être analysées avec un cytomètre de flux FACScan (Becton Dickinson). On observe que tous les surnageants NIH3T3 testés (résultant de l'infection avec les 3 types de particules virales) sécrètent un anticorps capable de se fixer sur la protéine cible GA733.

Dans le cas des constructions où l'anticorps est fusionné à la barnase ou à la version atténuée de celle-ci (pTG6356 et pTG6357), il est intéressant de pouvoir vérifier que l'anticorps fusionné a une activité nucléasique. Pour cela, on suit la dégradation d'un ARNt. 100 µl de surnageants à tester ou d'une solution de RNAseA à concentration connue (à titre de témoin de réaction) sont ajoutés à 200 µl de 0,5 M Tris-HCl pH 7,5, 5 mM EDTA, 0,5 mg/mg de BSA et 1 mg/ml final d'ARNt et incubés à 37°C pendant 30 min. Les tubes sont alors placés sur glace et 700 µl d'acide perchlorique à 6 % sont ajoutés pour précipiter l'ARNt pendant 10 min sur glace. Une centrifugation de 10 min à vitesse maximale à 4°C permet de culoter l'ARNt, laissant en suspension les nucléotides libres libérés par l'action de l'enzyme. L'absorbance de ces nucléotides est alors lue à 260 nm.

L'implant peut être constitué selon la méthode indiquée à l'exemple 1 par incorporation de fibroblastes murins primaires ou de cellules NIH3T3 transduits avec les vecteurs pTCT63S5, pTG6356 ou pTG6357.

### Exemple 3 : Préparation d'un implant secrétant une immunoadhésine et destiné à une immunothérapie anti-SIDA

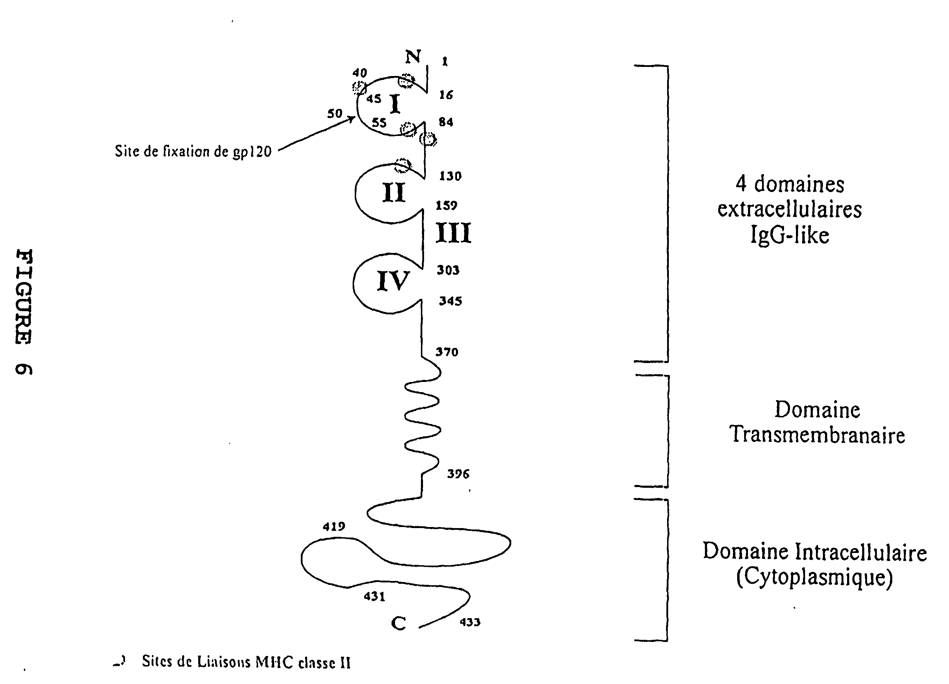

Il s'agit ici de produire une immunoadhésine résultant de la fusion d'une molécule "adhésive" liant la glycoprotéine du virus VIH et d'une immunoglobuline stabilisant la structure et conférant une immunité non spécifique. La partie adhésive dérive de la protéine membranaire CD4 (structure représentée à la Figure 6) dont on retient la partie N-terminale (séquence signal et domaines I et II de la région extracellulaire). Plusieurs études ont montré qu'ils sont capables à eux seuls de lier la gp120, de bloquer l'interaction et la pénétration du VIH dans les cellules cibles CD4⁺ (Traunecker et al., 1988, Nature 331, 84-86 ; Deen et al., 1988, Nature *331*, 82-84 ; Hussey et al., 1988, Nature *331*, 78-81 ; Fisher et al., 1988, Nature *331*, 76-78). La partie immunoglobuline est constituée par la région γ3 constante (région charnière - CH2-CH3) de l'anticorps 2F5. La molécule hybride est désignée par la suite sCD4-2F5.

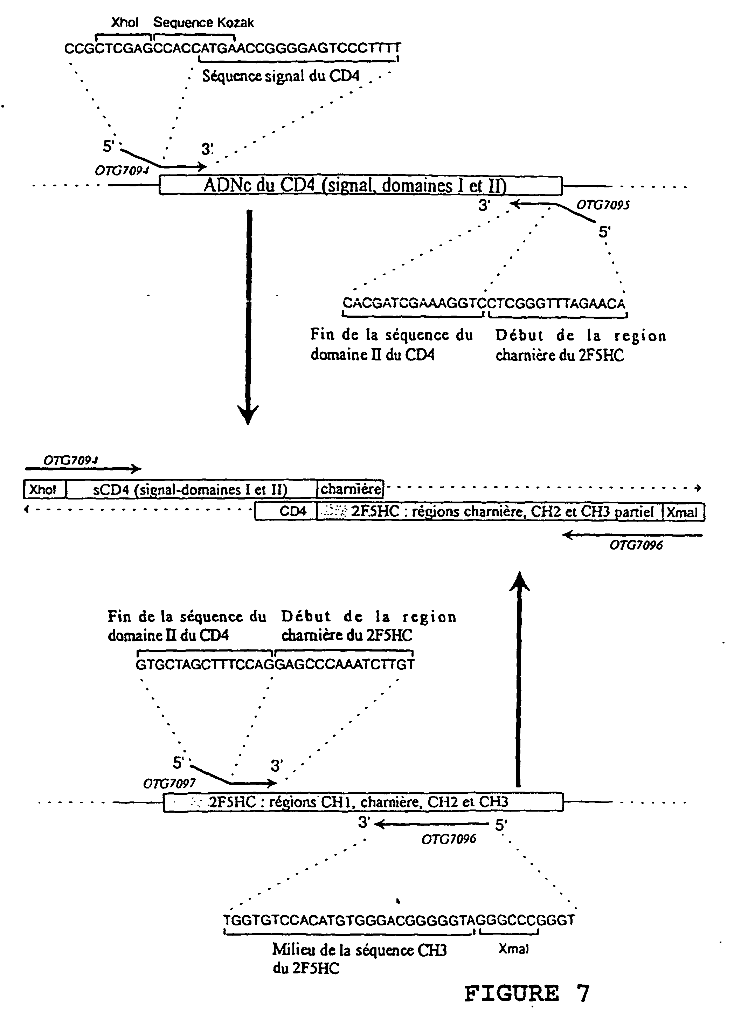

La construction est réalisée de la manière suivante (voir Figure 7) :

Les séquences codant pour la région sCD4 (séquence signal - domaines I et II) sont isolées classiquement par PCR. On utilise à titre de matrice l'ADNc CD4 décrit dans la littérature (obtenu à partir d'ARNm de cellules CD4⁺) ou un plasmide de l'art antérieur dans lequel l'ADNc est cloné (Jay Maddon et al., 1985, Cell *42*, 93-104), que l'on hybride aux amorces OTG7094 et OTG7095 (SEQ ID NO: 13 et 14). La première permet d'introduire un site *Xho*I et des séquences consensus de type Kozak en amont de l'ATG initiateur de CD4 et la seconde porte des nucléotides correspondant d'une part à l'extrémité C-terminale du domaine II CD4 et, d'autre part, à l'extrémité N-terminale de la région charnière du 2F5 HC. La réaction se déroule sur 25 cycles (1 min à 94°C, 2 min à 50°C et 3 min à 72°C).

Les séquences codant pour le segment γ3 constant du 2F5 HC sont également amplifiées par PCR. On met en oeuvre le plasmide pTG2677 et les amorces OTG7097 et OTG7096 (SEQ ID NO: 15 et 16). La première est complémentaire à OTG7095 et la seconde recouvre le site *Xma*I situé au sein de la région CH3.

Les produits PCR ainsi générés se recouvrent sur 30 pb. Ils sont rehybridés et soumis à une seconde réaction d'amplification qui se déroule en 2 étapes, en premier lieu, une amplification linéaire pour étendre le produit rehybridé (10 cycles : 1 min à 94°C, 2 min à 37°C et 3 min à 72°C) suivie d'une amplification exponentielle en présence des amorces OTG7094 et OTG7096 (SEQ ID NO:13 et 16) (20 cycles : 1 min à 94°C, 2 min à 50°C et 3 min à 72°C).

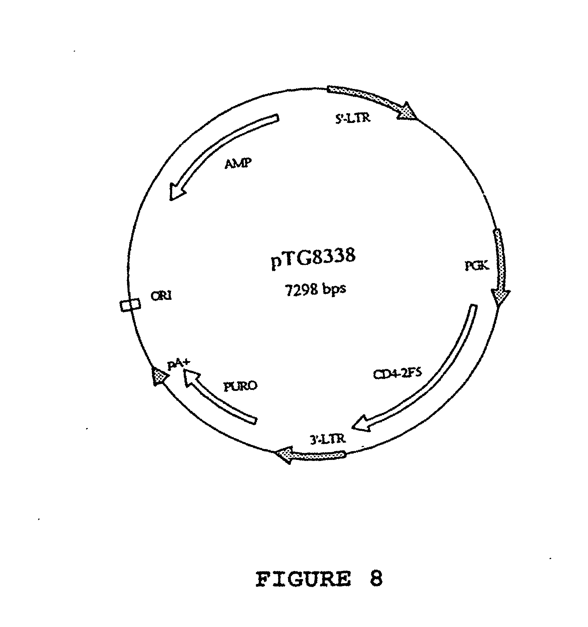

Le produit final est inséré entre les sites *Xho*I et *Xma*I de pTG2677 pour donner pTG8332 afin de reconstituer la molécule sCD4-2F5 complète. Celle-ci est excisée par digestion *Xho*I*-Bam*HI et clonée en aval du promoteur PGK murin dans le vecteur pTG6368. On obtient pTG8338 (Figure 8).

Les cellules NIH3T3 sont transfectées par 10 µg de pTG8338 linéarisé par *Bgl*II*.* Deux jours après, les cellules sont cultivées en concentration croissante de puromycine (5 à 75 µg/ml). L'expression de la protéine sCD4-2F5 est vérifiée par immunofluorescence en mettant en oeuvre un anticorps qui reconnaît soit la partie CD4 (Leu3A ; Becton Dickinson) soit la partie 2F5 (anticorps monoclonal de souris anti-région charnière d'une IgG3 humaine ; Interchim) et un conjugué constitué par un anticorps d'âne anti Ig de souris couplé à la fluorescine (Jackson Laboratories). On note qu'environ 30% du pool cellulaire exprime un niveau detectable d'immunoadhésine. Pour cette raison, on isole par dilution clonale des clones producteurs.

La production des particules virales est effectuée dans les cellules GP+E-86 transfectées selon le protocole classique et sélectionnées en présence de puromycine. Les cellules cibles NIH 3T3 sont ensuite infectées par le surnageant cellulaire et les analyses d'imunofluorescence confirment l'expression du transgène dans 100% des cellules.

La quantification est réalisée par ELISA. En premier lieu, on dépose 500 ng de glycoprotéine d'enveloppe gp160 du virus VIH-1 pour fixer la partie CD4 de la molécule CD4-2F5. Elle est produite par voie recombinante ainsi qu'indiqué dans la demande internationale WO92/19742. Puis, on ajoute le surnageant à doser et, enfin, un anticorps dirigé contre la partie 2F5 (anticorps de chèvre anti-Ig humaine conjugué à la péroxydase ; Interchim). La solution d'étalonnage est constitutée par l'immunoadhésine recombinante purifiée des surnageants de culture sur colonne de sépharose-protéine G à haut débit (Pharmacia). On mesure une productivité supérieure à 10 µg/ml/24 h/10⁶ cellules dans les surnageants des cellules NIH 3T3 infectées. Ce test indique également que la protéine est capable de fixer la gp160 et, par conséquent, serait apte à lier le virus VIH afin d'exercer sa fonction thérapeutique.

Les cellules NIH3T3 infectées sont amplifiées par culture dans les flasques F175. 4 organoïdes contenant chacun environ 10⁷ cellules sont générés en appliquant la technologie détaillée à l'exemple 1 et greffés dans la cavité péritonale de 4 souris femelles BALB/c nude. La sécrétion de l'immunoadhésine dans le sérum est suivie jusqu'à 5 semaines post-implantation (dosage par ELISA). Les résultats indiquent une concentration de l'ordre de 100 à 200 µg/ml et une sécrétion continue pendant la durée de l'expérience.

### Exemple 4 : Préparation d'un implant secrétant une protéine résultant de la fusion de l'immunoadhésine sCD4-2F5 et de l'angiogénine humaine

L'angiogénine est une protéine plasmatique de 14,1 kDa appartenant à la famille des enzymes ribonucléolytiques. Cependant, son action lytique est plus limitée que celle de la ribonuclease A de référence (RNase A) et montre une préférence marquée pour certains ARNs (notamment pour les ARNs ribosomaux 18S et 28S et les ARNs de transfert). Le gène et l'ADNc ont été clonés il y a déjà une dizaine d'années (Kurachi et al., 1985, Biochemistry 74, 5494-5499).

La fusion des séquences codant pour l'angiogénine en aval de sCD4-2F5 devrait permettre la synthèse d'une protéine capable de cibler et détruire les cellules infectées par le VIH. Celles-ci ont été obtenues par PCR du plasmide pHAG1 (Kurachi et al., 1985, *supra*) à l'aide des amorces OTG10089 et OTG10090 (SEQ ID NO: 17 et 18) comportant à leurs extrémités 5' les sites de restriction *Eco*RI et *Bam*HI situés respectivement en 5' du premier codon de la protéine mature et en 3' du codon stop.

Pour des raisons d'encombrement stérique, on choisit d'introduire un espaceur entre les deux entités. La réaction PCR est effectuée à l'aide de la matrice pTG2677 et des oligonucléotides OTG10087 et OTG10088 (SEQ ID NO: 19 et 20) afin d'amplifier la partie du gène 2F5 s'étendant du site *Xma*I (au sein de CH3) au codon stop. L'amorce OTG10088 est conçue pour éliminer le codon stop et introduire en 3' un site *Bam*HI ainsi que l'espaceur codant pour les résidus Gly-Gly-Gly-Gly-Ser.

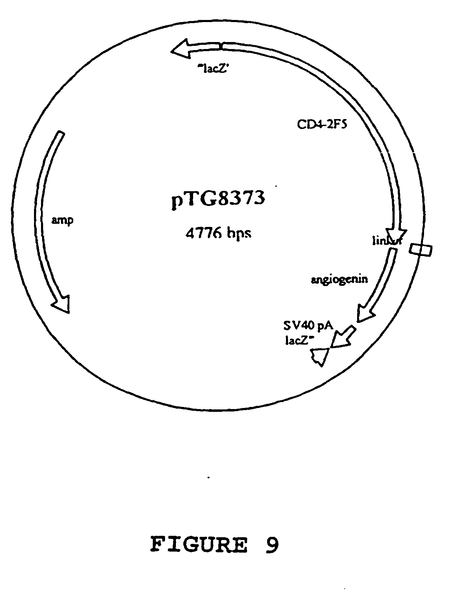

Les deux fragments PCR obtenus, bordés par un site *Bam*HI sont ligués ensembles. On isole le fragment *Xma*I*-Eco*RI*,* lequel est inséré tout d'abord dans le pBluescript afin de vérifier la séquence et, enfin dans le vecteur pTG8332 pour reconstituer la séquence de fusion complète "sCD4-2F5-Angiogénine". On obtient pTG8373 (Figure 9). Le bloc complet peut être excisé par digestion *Xho*I*-Bgl*II et cloné dans le vecteur retroviral pTG6368 linéarisé par *Xho*I et *Bam*HI*.* Les particules virales peuvent être constituées comme précédemment et les organoïdes générés à partir de cellules cibles infectées NIH 3T3 ou fibroblastes primaires.

### Exemple 5 : Préparation d'un vecteur adénoviral exprimant l'immunoadhésine sCD4-2F5

Les fragments de génome adénoviral employés dans les constructions décrites ci-après, sont indiqués précisemment selon leur position dans la séquence nucléotidique du génome de l'adénovirus de type 5 (Ad5) telle que divulguée dans la banque de données Genebank sous la référence M73260.

L'intron et le signal de polyadénylation (pA) du gène humain de la β-globine sont obtenus du vecteur pBCMG/Néo (Karasuyama, 1988, Eur J. Immuno. *18*, 97-104 ; Karasuyama, 1989, J. Exp. Med. *169,* 13-25) et introduits dans le plasmide pREP4 (In Vitogen™) en aval du LTR 3' du virus RSV. La cassette "promoteur RSV-intron-pA β-globine" est isolée du vecteur précédent sous forme d'un fragment *Sal*I*-Bam*HI et insérée dans le vecteur pTG9350. Ce dernier provient du clonage des séquences génomiques de l'Ad5 s'étendant des nucléotides 1 à 458 et 3328 à 5788 dans le p polyII (Lathe et al., 1987,Gene 57, 193-201).

On introduit dans le vecteur pTG8346 obtenu à l'étape précédente (entre l'intron et le pA) un poly linker pourvu de sites multiples de clonage (*Eco*RI, *Xho*I*, Not*I*, Xba*I, *Spe*I, *Bam*HI, *Eco*RV*, Hin*dIII, *Cla*I, *Kpn*I et *Bgl*II), pour créér pTG8347.

Les séquences codant pour la protéine hybride sCD4-2F5 sont isolées du vecteur pTG8338 (Exemple 3) par digestion *Xho*I *Bam*HI et introduites dans le vecteur pTG8347 clivé par *Xho*I et *Bgl*III, pour générer pTG8349 lequel permet leur expression sous le contrôle du promoteur RSV.

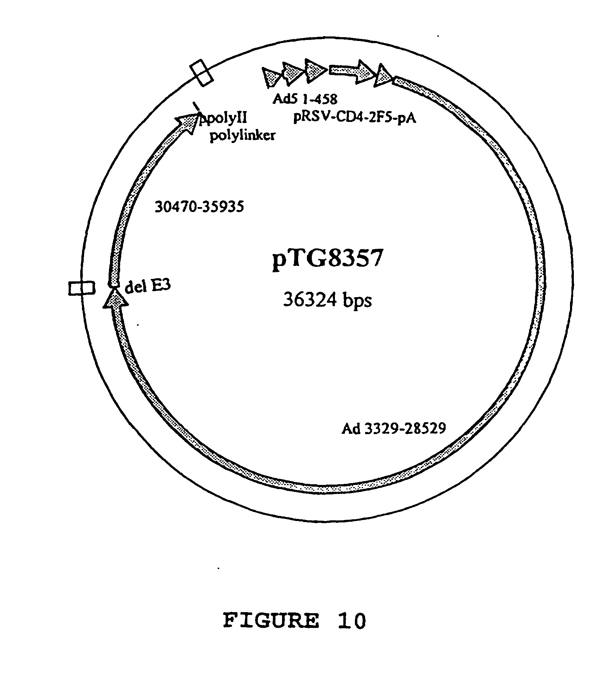

On utilise la technique de recombinaison homologue *in vitro* (décrite dans la demande française 94 14470) pour reconstituer le génome complet du vecteur recombinant. On met en oeuvre à cet effet, le vecteur pTG4656 qui comprend le génome de l'Ad5 délété des régions E1 et E3 (Ad5 1 à 458 - promoteur MLP Ad2 - gène LacZ - pA SV40 - Ad5 3329 à 28529 et 30470 à 35935). Tout autre vecteur adénoviral E1⁻ E3⁻ peut également convenir, comme ceux décrits dans la demande internationale WO94/28152. Les cellules BJ5183 (Hanahan, 1983, J. Mol. Biol. *166,* 557-580) sont co-transformées par pTG4656 linéarisé par l'enzyme *Cla*I (10 à 20 ng) et le fragment *Pac*I*-Bst*XI purifié de pTG8349 (excès molaire de 10 fois environ). La recombinaison au niveau des séquences adénovirales homologues entraîne le remplacement de la cassette LacZ de pTG4656 par celle de sCD4-2F5 (Promoteur RSV - intron β-globine - gène sCD4-2F5 - pA β-globine) portée le fragment issu de pTG8349. On génère le vecteur pTG8357 (Figure 10).

Les virus recombinants sont obtenus par transfection de pTG8357 dans les cellules 293 (ATCC CRL1573). On sélectionne 5 plages qui sont amplifiées par culture dans des flasques F25 en présence de cellules 293 fraîches. Après 5 jours, les cellules infectées sont récoltées et soumises à une analyse de HIRTH (Gluzman et Van Doren, 1983, J. Virol. *45,* 91-103), afin de vérifier la présence du transgène. Brièvement, l'analyse de HIRTH consiste en une extraction du génome adénoviral, une précipitation de l'ADN viral, une digestion avec une enzyme de restriction appropriée, un transfert sur membrane et une hybridation avec une sonde radioactive capable de s'hybrider aux séquences sCD4-2F5. On observe un signal positif pour les 5 adénovirus analysés.

Un stock adénoviral conséquent est constitué par amplification successive dans les cellules 293, purification sur deux gradients de chlorure de césium et dialyse. Ce stock peut être utilisé dans le cadre d'essais cliniques anti-SIDA.

### Exemple 6 : Préparation d'un vecteur adénoviral exprimant l'immunoadhésine cytotoxique sCD4-2F5-angiogénine

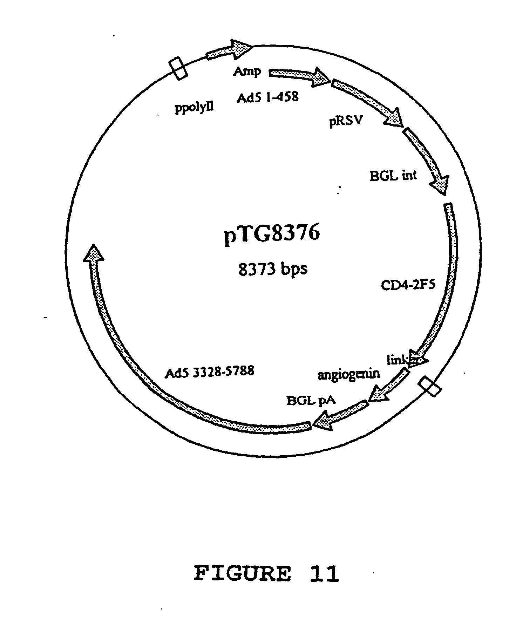

Les séquences codant pour la protéine de fusion sCD4-2F5-angiogénine sont excisées du vecteur pTG8373 (Exemple 4) par digestion *Xho*I*-Bgl*II et clonées au niveau des mêmes sites dans le vecteur pTG8347 (Exemple 5). On obtient le vecteur pTG8376 (Figure 11) qui peut être soumis à la recombinaison homologue avec un vecteur adénoviral, par exemple pTG4656, pour produire les adénovirus recombinants défectifs exprimant l'immunoadhésine cytotoxique dirigée contre les cellules infectées par le virus VIH.

## Revendications

1. Un implant de cellules génétiquement modifiées comprenant une séquence nucléotidique exogène codant pour tout ou partie d'un anticorps dirigé contre un antigène tumoral ou un épitope spécifique d'un virus, ladite séquence nucléotidique exogène étant placée sous le contrôle des éléments nécessaires à son expression et à la secrétion dudit anticorps, lesdites cellules étant fixées à une matrice extracellulaire et ledit anticorps sécrété par ledit implant étant capable de lier spécifiquement ledit antigène ou ledit épitope contre lequel il est dirigé.

2. Un implant selon la revendication 1, **caractérisé en ce que** ledit anticorps est sélectionné parmi le groupe constitué par :
- un anticorps natif,
- un anticorps chimérique,
- un fragment d'anticorps, et notamment un fragment Fab, F(ab')₂, ou encore scFv, et
- un anticorps bispécifique.

3. Un implant selon la revendication 1 ou 2, **caractérisé en ce que** ledit anticorps est modifié par fusion à une substance toxique ou immunopotentiatrice.

4. Un implant selon la revendication 3, **caractérisé en ce que** ladite substance toxique est sélectionnée parmi une ribonucléase, et notamment la ribonucléase de *Bacillus amyloliquefaciens,* la ricine, la toxine diphtérique, la toxine cholérique, la thymidine kinase du virus simplex de l'herpès, la cytosine déaminase d*'Escherichia coli* ou d'une levure du genre *Saccharomyces,* l'exotoxine de *Pseudomonas* et l'angiogénine humaine ou un analogue desdites substances.

5. Un implant selon l'une des revendications 1 à 4, **caractérisé en ce que** ladite séquence nucléotidique exogène placée sous le contrôle des éléments nécessaires à son expression et à !a sécrétion dudit anticorps est portée par un vecteur dérivé d'un plasmide, d'un rétrovirus, d'un virus de l'herpès, d'un adénovirus ou d'un virus associé à l'adénovirus, ledit vecteur étant introduit dans lesdites cellules génétiquement modifiées par transfection.

6. Un implant selon la revendication 5, **caractérisé en ce que** ledit vecteur est dicistronique.

7. Un implant selon la revendication 6, **caractérisé en ce que** ledit vecteur est rétroviral et comprend de 5' vers 3' :
(a) un LTR 5' dérivé d'un rétrovirus,
(b) une région d'encapsidation,
(c) une séquence nucléotidique exogène comprenant :
- un promoteur interne,
- une première séquence codant pour la chaîne lourde d'un anticorps,
- un site d'initiation de l'entrée des ribosomes,
- une deuxième séquence codant pour la chaîne légère d'un anticorps, et
(d) un LTR 3' dérivé d'un rétrovirus.

8. Un implant selon la revendication 7, **caractérisé en ce que** ladite séquence nucléotidique exogène comprend en outre une troisième séquence codant pour une substance toxique ou immunopotentiatrice fusionnée en aval et de manière opérationnelle à la deuxième séquence.

9. Un implant selon l'une des revendications 1 à 8, comprenant des cellules autologues génétiquement modifiées.

10. Un implant selon la revendication 9, comprenant des fibroblastes génétiquement modifiés.

11. Un implant selon l'une des revendications 1 à 10, **caractérisé en ce qu'**il comprend de 10⁶ à 10¹², de préférence de 10⁷ à 10¹¹ cellules génétiquement modifiées.

12. Méthode de préparation d'un implant selon l'une des revendications 1 à 11, **caractérisée en ce que** l'on met en présence les cellules génétiquement modifiées et une matrice extracellulaire.

13. Utilisation d'un implant selon l'une des revendications 1 à 11, pour la préparation d'une composition pharmaceutique destinée au traitement ou à la prévention d'une maladie acquise.

14. Utilisation d'un implant selon la revendication 13, pour la préparation d'une composition pharmaceutique destinée au traitement ou à la prévention d'une maladie infectieuse, et notamment du SIDA, ou du cancer.

15. Un vecteur adénoviral recombinant comprenant une séquence nucléotidique exogène codant pour tout ou partie d'un anticorps dirigé contre un antigène tumoral ou un épitope spécifique d'un microorganisme infectieux et pathogène et modifié par fusion à une substance toxique ou immunopotentiatrice, ladite séquence nucléotidique étant placée sous le contrôle des éléments nécessaires à son expression et ledit anticorps étant capable de lier spécifiquement ledit antigène ou ledit épitope contre lequel il est dirigé.

16. Un vecteur adénoviral recombinant selon la revendication 15, **caractérisé en ce que** ledit anticorps est sélectionné parmi le groupe constitué par un anticorps natif, un anticorps chimérique, un fragment d'anticorps et, notamment, un fragment F(ab')₂, Fc ou encore scFv et un anticorps bispécifique.

17. Un vecteur adénoviral recombinant selon la revendication 15, **caractérisé en ce que** ladite substance toxique est sélectionnée parmi une ribonucléase, et notamment la ribonucléase de *Bacillus amyloliquefaciens,* la ricine, la toxine diphtérique, la toxine cholérique, la thymidine kinase du virus simplex de l'herpès, la cytosine déaminase d'*Escherichia coli* ou d'une levure du genre *Saccharomyces,* l'exotoxine de *Pseudomonas* et l'angiogénine humaine ou un analogue desdites substances.

18. Un vecteur adénoviral recombinant selon la revendication 15, **caractérisé en ce que** ledit anticorps est modifié par fusion à une substance immunopotentiatrice.

19. Un vecteur adénoviral recombinant comprenant une séquence nucléotidique exogène codant pour tout ou partie d'une ou plusieurs protéine(s) d'intérêt capable(s) de former un multimère, tel qu'un dimère ou un tétramère, dans une cellule hôte ; ladite séquence nucléotidique exogène étant placée sous le contrôle des éléments nécessaires à son expression, ledit vecteur étant dérivé d'un adénovirus d'origine humaine, canine, aviaire, bovine, murine, ovine, porcine ou simienne ou encore d'un hybride comprenant des fragments de génome adénoviral de différentes origines et ledit vecteur étant **caractérisé par le fait que** ladite séquence nucléotidique exogène code pour tout ou partie d'au moins un anticorps dirigé contre un antigène tumoral ou un épitope spécifique d'un microorganisme infectieux et pathogène, ledit anticorps étant capable de lier spécifiquement ledit antigène ou ledit épitope contre lequel il est dirigé.

20. Un vecteur adénoviral recombinant selon la revendication 19, **caractérisé par le fait que** ledit anticorps est modifié par fusion à une substance toxique ou immunopotentiatrice.

21. Un vecteur adénoviral recombinant selon l'une des revendications 15 à 18, dérivé d'un adénovirus d'origine humaine, canine, aviaire, bovine, murine, ovine, porcine ou simienne ou encore d'un hybride comprenant des fragments de génome adénoviral de différentes origines.

22. Un vecteur adénoviral recombinant selon l'une des revendications 15 à 21, **caractérisé en ce qu'**il est défectif pour la replication.

23. Un vecteur adénoviral recombinant selon la revendication 22, **caractérisé en ce qu'**il est au moins dépourvu de tout ou partie de la région E1 et, de façon optionnelle, de tout ou partie de la région E3.

24. Un vecteur adénoviral recombinant selon la revendication 22 ou 23, comprenant une séquence nucléotidique exogène codant pour la chaîne lourde de l'anticorps 2F5, un élément IRES et la chaîne légère de l'anticorps 2F5 ; ladite séquence nucléotidique exogène étant placée sous le contrôle des éléments nécessaires à son expression.

25. Un vecteur adénoviral recombinant selon la revendication 22 ou 23, comprenant une séquence nucléotidique exogène codant pour la séquence signal et les domaines 1 et II extracellulaires de la protéine CD4 fusionnés de manière opérationnelle à la région γ3 constante (région charnière-CH2 et CH3) de la chaîne lourde de l'anticorps 2F5.

26. Un vecteur adénoviral recombinant selon la revendication 22 ou 23, comprenant une séquence nucléotidique exogène codant pour la séquence signal et les domaines I et II extracellulaires de la protéine CD4 fusionnés de manière opérationnelle à la région γ3 constante (région charnière-CH2 et CH3) de la chaîne lourde de l'anticorps 2F5 et fusionnée de manière opérationnelle à l'angiogénine humaine mature.

27. Un vecteur adénoviral recombinant selon l'une des revendications 15 à 26, **caractérisé en ce que** les éléments nécessaires à l'expression comprennent un promoteur sélectionné parmi le groupe constitué par le promoteur précoce adénoviral EIA, le promoteur tardif MLP (Major Late Promoter), le promoteur murin ou humain PGK (Phosphoglycérate kinase), le promoteur précoce du virus SV40, le promoteur du virus RSV (Rous Sarcoma virus), un promoteur actif spécifiquement dans les cellules tumorales et enfin un promoteur actif spécifiquement dans les cellules infectées.

28. Une particule virale infectieuse comprenant un vecteur adénoviral recombinant selon l'une des revendications 15 à 27.

29. Une cellule hôte eucaryote comprenant un vecteur adénoviral recombinant selon l'une des revendications 15 à 27 ou une particule virale infectieuse selon la revendication 28.

30. Une composition pharmaceutique comprenant un vecteur adénoviral recombinant selon l'une des revendications 15 à 27, une particule virale infectieuse selon la revendication 28 ou une cellule hôte eucaryote selon la revendication 29, en association avec un support acceptable d'un point de vue pharmaceutique.

31. Une composition pharmaceutique selon la revendication 30, comprenant 10⁴ à 10¹⁴ pfu.

32. Une composition pharmaceutique selon la revendication 30 ou 31, **caractérisée en ce qu'**elle est sous forme injectable.

33. Utilisation d'un vecteur adénoviral recombinant selon l'une des revendications 15 à 27, d'une particule virale infectieuse selon la revendication 28 ou d'une cellule hôte eucaryote selon la revendication 29 pour la préparation d'une composition pharmaceutique destinée au traitement et/ou à la prévention du corps humain ou animal par thérapie génique.

34. Utilisation selon la revendication 33, pour la préparation d'une composition pharmaceutique destinée au traitement et/ou à la prévention des maladies acquises et, notamment des cancers et du SIDA.

35. Utilisation selon la revendication 34, pour la préparation d'une composition pharmaceutique administrable par voie intraveineuse ou intratumorale.

## Patentansprüche

1. Implantat von genetisch modifizierten Zellen, die eine exogene Nukleotidsequenz umfassen, welche für das Ganze oder einen Teil eines Antikörpers kodiert, der gegen ein tumorales Antigen oder ein spezifisches Epitop eines Virus gerichtet ist, wobei die exogene Nukleotidsequenz unter die Kontrolle von Elementen gestellt ist, die für ihre Expression und die Sekretion des Antikörpers erforderlich sind, wobei die Zellen an einer extrazellulären Matrix befestigt sind und der von dem Implantat sekretierte Antikörper in der Lage ist, spezifisch das Antigen oder das Epitop, gegen das er gerichtet ist, zu binden.

2. Implantat nach Anspruch 1, **dadurch gekennzeichnet, dass** der Antikörper ausgewählt ist aus der Gruppe, die besteht aus:
- einem nativen Antikörper,
- einem chimären Antikörper,
- einem Antikörper-Fragment und insbesondere einem Fab-, F(ab')₂- oder auch scFv-Fragment und
- einem bispezifischen Antikörper.

3. Implantat nach Anspruch 1 oder 2, **dadurch gekennzeichnet, dass** der Antikörper durch Fusion mit einer toxischen oder immunpotenzierenden Substanz modifiziert ist.

4. Implantat nach Anspruch 3, **dadurch gekennzeichnet, dass** die toxische Substanz ausgewählt ist aus einer Ribonuklease und insbesondere der Ribonuklease von *Bacillus amyloliquefaciens,* Ricin, Diphterie-Toxin, Cholera-Toxin, Thymidin-Kinase des Herpes-simplex-Virus, Cytosin-Desaminase von *Escherichia coli* oder einer Hefe der Gattung *Saccharomyces,* dem Exotoxin von *Pseudomonas* und dem menschlichen Angiogenin oder einem Analogon dieser Substanzen.

5. Implantat nach einem der Ansprüche 1 bis 4, **dadurch gekennzeichnet, dass** die exogene Nukleotidsequenz, die unter die Kontrolle der Elemente gestellt ist, die für ihre Expression und die Sekretion des Antikörpers erforderlich sind, von einem Vektor getragen wird, der von einem Plasmid, einem Retrovirus, einem Herpes-Virus, einem Adenovirus oder einem Adenovirusassoziierten Virus abgeleitet ist, wobei der Vektor durch Transfektion in die genetisch modifizierten Zellen eingeführt wird.

6. Implantat nach Anspruch 5, **dadurch gekennzeichnet, dass** der Vektor dicistronisch ist.

7. Implantat nach Anspruch 6, **dadurch gekennzeichnet, dass** der Vektor retroviral ist und von 5' in Richtung 3' umfasst:
(a) ein 5'-LTR, das von einem Retrovirus abgeleitet ist,
(b) eine Enkapsidierungsregion,
(c) eine exogene Nukleotidsequenz, welche umfasst:
- einen internen Promotor,
- eine erste Sequenz, welche für die schwere Kette eines Antikörpers kodiert,
- eine Initiationsstelle des Eingangs der Ribosomen,
- eine zweite Sequenz, welche für die leichte Kette eines Antikörpers kodiert, und
(d) ein 3'-LTR, das von einem Retrovirus abgeleitet ist.

8. Implantat nach Anspruch 7, **dadurch gekennzeichnet, dass** die exogene Nukleotidsequenz darüber hinaus eine dritte Sequenz umfasst, die für eine toxische oder immunpotenzierende Substanz kodiert und stromabwärts und operativ mit der zweiten Sequenz fusioniert ist.

9. Implantat nach einem der Ansprüche 1 bis 8, das autologe genetisch modifizierte Zellen umfasst.

10. Implantat nach Anspruch 9, das genetisch modifizierte Fibroblasten umfasst.

11. Implantat nach einem der Ansprüche 1 bis 10, **dadurch gekennzeichnet, dass** es 10⁶ bis 10¹², bevorzugt 10⁷ bis 10¹¹ genetisch modifizierte Zellen umfasst.

12. Verfahren zur Herstellung eines Implantats nach einem der Ansprüche 1 bis 11, **dadurch gekennzeichnet, dass** man genetisch modifizierte Zellen und eine extrazelluläre Matrix in Kontakt bringt.

13. Verwendung eines Implantats nach einem der Ansprüche 1 bis 11 für die Herstellung einer pharmazeutischen Zusammensetzung, die zur Behandlung oder Verhütung einer erworbenen Krankheit bestimmt ist.

14. Verwendung eines Implantats nach Anspruch 13 für die Herstellung einer pharmazeutischen Zusammensetzung, die zur Behandlung oder Verhütung einer infektiösen Krankheit und insbesondere von AIDS oder von Krebs bestimmt ist.

15. Rekombinanter adenoviraler Vektor, umfassend eine exogene Nukleotidsequenz, die für das Ganze oder einen Teil eines Antikörpers kodiert, der gegen ein tumorales Antigen oder ein spezifisches Epitop eines infektiösen und pathogenen Mikroorganismus gerichtet ist und durch Fusion mit einer toxischen oder immunpotenzierenden Substanz modifiziert ist, wobei die Nukleotidsequenz unter die Kontrolle von Elementen gestellt ist, die für ihre Expression erforderlich sind, und der Antikörper in der Lage ist, spezifisch das Antigen oder das Epitop, gegen das er gerichtet ist, zu binden.

16. Rekombinanter adenoviraler Vektor nach Anspruch 15, **dadurch gekennzeichnet, dass** der Antikörper aus der Gruppe ausgewählt ist, die aus einem nativen Antikörper, einem chimären Antikörper, einem Antikörper-Fragment und insbesondere einem F(ab')₂-, Fc- oder auch scFv-Fragment und einem bispezifischen Antikörper besteht.

17. Rekombinanter adenoviraler Vektor nach Anspruch 15, **dadurch gekennzeichnet, dass** die toxische Substanz aus einer Ribonuklease und insbesondere der Ribonuklease von *Bacillus amyloliquefaciens,* Ricin, Diphterie-Toxin, Chotera-Toxin, Thymidin-Kinase des Herpes-simplex-Virus, Cytosin-Desaminase von *Escherichia coli* oder einer Hefe der Gattung *Saccharomyces,* dem Exotoxin von *Pseudomonas* und dem menschlichen Angiogenin oder einem Analogon dieser Substanzen ausgewählt ist.

18. Rekombinanter adenoviraler Vektor nach Anspruch 15, **dadurch gekennzeichnet, dass** der Antikörper durch Fusion mit einer immunpotenzierenden Substanz modifiziert ist.

19. Rekombinanter adenoviraler Vektor, umfassend eine exogene Nukleotidsequenz, die für das Ganze oder einen Teil eines oder mehrerer interessierender Proteine kodiert, das bzw. die in der Lage ist/sind, ein Multimer, wie ein Dimer oder ein Tetramer, in einer Wirtszelle zu bilden; wobei die exogene Nukleotidsequenz unter die Kontrolle von Elementen gestellt ist, die für ihre Expression erforderlich sind, wobei der Vektor von einem Adenovirus humanen, caninen, avianen, bovinen, murinen, ovinen, porcinen oder simianen Ursprungs oder auch von einem Hybrid abgeleitet ist, das Fragmente von adenoviralem Genom verschiedener Ursprünge umfasst, und wobei der Vektor **dadurch gekennzeichnet ist, dass** die exogene Nukleotidsequenz für das Ganze oder einen Teil mindestens eines Antikörpers kodiert, der gegen ein tumorales Antigen oder ein spezifisches Epitop eines infektiösen und pathogenen Mikroorganismus gerichtet ist, wobei der Antikörper in der Lage ist, spezifisch das Antigen oder das Epitop, gegen das er gerichtet ist, zu binden.

20. Rekombinanter adenoviraler Vektor nach Anspruch 19, **dadurch gekennzeichnet, dass** der Antikörper durch Fusion mit einer toxischen oder immunpotenzierenden Substanz modifiziert ist.

21. Rekombinanter adenoviraler Vektor nach einem der Ansprüche 15 bis 18, der von einem Adenovirus humanen, caninen, avianen, bovinen, murinen, ovinen, porcinen oder simianen Ursprungs oder auch von einem Hybrid abgeleitet ist, das Fragmente von adenoviralem Genom verschiedener Ursprünge umfasst.

22. Rekombinanter adenoviraler Vektor nach einem der Ansprüche 15 bis 21, **dadurch gekennzeichnet, dass** er replikationsdefizient ist.

23. Rekombinanter adenoviraler Vektor nach Anspruch 22, **dadurch gekennzeichnet, dass** ihm mindestens das Ganze oder ein Teil der Region E1 und gegebenenfalls das Ganze oder ein Teil der Region E3 fehlt.

24. Rekombinanter adenoviraler Vektor nach Anspruch 22 oder 23, umfassend eine exogene Nukleotidsequenz, die für die schwere Kette des Antikörpers 2F5, ein IRES-Element und die leichte Kette des Antikörpers 2F5 kodiert; wobei die exogene Nukleotidsequenz unter die Kontrolle von Elementen gestellt ist, die für ihre Expression erforderlich sind.

25. Rekombinanter adenoviraler Vektor nach Anspruch 22 oder 23, umfassend eine exogene Nukleotidsequenz, welche für die Signalsequenz und die extrazellulären Domänen I und II des CD4-Proteins kodiert, die operativ mit der γ3 konstanten Region (CH2-Scharnierregion und CH3) der schweren Kette des Antikörpers 2F5 fusioniert sind.

26. Rekombinanter adenoviraler Vektor nach Anspruch 22 oder 23, umfassend eine exogene Nukleotidsequenz, welche für die Signalsequenz und die extrazellulären Domänen I und II des CD4-Proteins kodiert, die operativ mit der γ3 konstanten Region (CH2-Scharnierregion und CH3) der schweren Kette des Antikörpers 2F5 fusioniert sind, und die operativ mit reifem menschlichem Angiogenin fusioniert ist.

27. Rekombinanter adenoviraler Vektor nach einem der Ansprüche 15 bis 26, **dadurch gekennzeichnet, dass** die für die Expression erforderlichen Elemente einen Promotor umfassen, der aus der Gruppe ausgewählt ist, die aus dem frühen adenoviralen E1A-Promotor, dem späten MLP-Promotor (Major Late Promoter), dem murinen oder humanen PGK- (Phosphoglycerat-Kinase-) Promotor, dem frühen Promotor des SV40-Virus, dem Promotor des RSV (Rous-Sarcoma-Virus), einem Promotor, der spezifisch in Tumorzellen aktiv ist, und schließlich einem Promotor, der spezifisch in infizierten Zellen aktiv ist, besteht.

28. Infektiöses virales Partikel, umfassend einen rekombinanten adenoviralen Vektor nach einem der Ansprüche 15 bis 27.

29. Eukaryotische Wirtszelle, umfassend einen rekombinanten adenoviralen Vektor nach einem der Ansprüche 15 bis 27 oder ein infektiöses virales Partikel nach Anspruch 28.

30. Pharmazeutische Zusammensetzung, umfassend einen rekombinanten adenoviralen Vektor nach einem der Ansprüche 15 bis 27, ein infektiöses virales Partikel nach Anspruch 28 oder eine eukaryotische Wirtszelle nach Anspruch 29 in Verbindung mit einem unter pharmazeutischem Gesichtspunkt annehmbaren Träger.

31. Pharmazeutische Zusammensetzung nach Anspruch 30, umfassend 10⁴ bis 10¹⁴ pfu.

32. Pharmazeutische Zusammensetzung nach Anspruch 30 oder 31, **dadurch gekennzeichnet, dass** sie in injizierbarer Form vorliegt.

33. Verwendung eines rekombinanten adenoviralen Vektors nach einem der Ansprüche 15 bis 27, eines infektiösen viralen Partikels nach Anspruch 28 oder einer eukaryotischen Wirtszelle nach Anspruch 29 für die Herstellung einer pharmazeutischen Zusammensetzung, die zur Behandlung und/oder Vorbeugung des menschlichen oder tierischen Körpers durch Gentherapie bestimmt ist.

34. Verwendung nach Anspruch 33 für die Herstellung einer pharmazeutischen Zusammensetzung, die zur Behandlung und/oder Verhütung von erworbenen Krankheiten und insbesondere von Krebsen und von AIDS bestimmt ist.

35. Verwendung nach Anspruch 34 für die Herstellung einer pharmazeutischen Zusammensetzung, die auf intravenösem oder intratumoralem Weg verabreichbar ist.

## Claims

1. Implant of genetically modified cells comprising an exogenous nucleotide sequence encoding all or part of an antibody directed against a tumour antigen or an epitope specific for a virus, the said exogenous nucleotide sequence being placed under the control of the elements necessary for its expression and for the secretion of the said antibody, the said cells being attached to an extracellular matrix and the said antibody secreted by the said implant being capable of specifically binding the said antigen or the said epitope against which it is directed.

2. Implant according to Claim 1, **characterized in that** the said antibody is selected from the group consisting of:
- a native antibody,
- a chimeric antibody,
- an antibody fragment, especially a fragment Fab, F(ab')₂ or scFv, and
- a bispecific antibody.

3. Implant according to Claim 1 or 2, **characterized in that** the said antibody is modified by fusion to a toxic or immunopotentiating substance.

4. Implant according to Claim 3, **characterized in that** the said toxic substance is selected from a ribonuclease, and especially the ribonuclease from *Bacillus amyloliquefaciens,* ricin, diphtheria toxin, cholera toxin, herpes simplex virus thymidine kinase, cytosine deaminase from *Escherichia coli* or from a yeast of the genus *Saccharomyces,* exotoxin from *Pseudomonas* and human angiogenin or an analogue of the said substances.

5. Implant according to one of Claims 1 to 4, **characterized in that** the said exogenous nucleotide sequence placed under the control of the elements necessary for its expression and for the secretion of the said antibody is carried by a vector derived from a plasmid, from a retrovirus, from a herpesvirus, from an adenovirus or from an adenovirus associated virus, the said vector being introduced into the said genetically modified cells by transfection.

6. Implant according to Claim 5, **characterized in that** the said vector is dicistronic.

7. Implant according to Claim 6, **characterized in that** the said vector is retroviral and comprises from 5' to 3':
(a) a 5' LTR derived from a retrovirus,
(b) an encapsidation region,
(c) an exogenous nucleotide sequence comprising:
- an internal promoter,
- a first sequence encoding the heavy chain of an antibody,
- a ribosome entry initiation site,
- a second sequence encoding the light chain of an antibody, and
(d) a 3' LTR derived from a retrovirus.

8. Implant according to Claim 7, **characterized in that** the said exogenous nucleotide sequence comprises, in addition, a third sequence encoding a toxic or immunopotentiating substance fused downstream and operably to the second sequence.

9. Implant according to one of Claims 1 to 8, comprising genetically modified autologous cells.

10. Implant according to Claim 9, comprising genetically modified fibroblasts.

11. Implant according to one of Claims 1 to 10, **characterized in that** it comprises from 10⁶ to 10¹², preferably from 10⁷ to 10¹¹ genetically modified cells.

12. Method for the preparation of an implant according to one of Claims 1 to 11, **characterized in that** the genetically modified cells and an extracellular matrix are placed in contact.

13. Use of an implant according to one of Claims 1 to 11, for the preparation of a pharmaceutical composition intended for the treatment or for the prevention of an acquired disease.

14. Use of an implant according to Claim 13, for the preparation of a pharmaceutical composition intended for the treatment or for the prevention of an infectious disease, and especially AIDS, or cancer.

15. Recombinant adenoviral vector comprising an exogenous nucleotide sequence encoding all or part of an antibody directed against a tumour antigen or an epitope specific for an infectious and pathogenic microorganism, and modified by fusion to a toxic or immunopotentiating substance, the said nucleotide sequence being placed under the control of the elements necessary for its expression and the said antibody being capable of specifically binding the said antigen or the said epitope against which it is directed.

16. Recombinant adenoviral vector according to Claim 15, **characterized in that** the said antibody is selected from the group consisting of a native antibody, a chimeric antibody, an antibody fragment and especially a fragment F(ab')₂, Fc or scFv and a bispecific antibody.

17. Recombinant adenoviral vector according to Claim 15, **characterized in that** the said toxic substance is selected from a ribonuclease, and especially the ribonuclease from *Bacillus amyloliquefaciens,* ricin, diphtheria toxin, cholera toxin, herpes simplex virus thymidine kinase, cytosine deaminase from *Escherichia coli* or from a yeast of the genus *Saccharomyces,* exotoxin from *Pseudomonas* and human angiogenin or an analogue of the said substances.

18. Recombinant adenoviral vector according to Claim 15, **characterized in that** the said antibody is modified by fusion to an immunopotentiating substance.

19. Recombinant adenoviral vector comprising an exogenous nucleotide sequence encoding all or part of one or more protein(s) of interest capable of forming a multimer, such as a dimer or a tetramer, in a host cell, the said exogenous nucleotide sequence being placed under the control of the elements necessary for its expression, the said vector being derived from an adenovirus of human, canine, avian, bovine, murine, ovine, porcine or simian origin or else from a hybrid comprising adenoviral genome fragments of different origins, and the said vector being **characterized in that** the said exogenous nucleotide sequence encodes all or part of at least one antibody directed against a tumour antigen or an epitope specific for an infectious and pathogenic microorganism, the said antibody being capable of specifically binding the said antigen or the said epitope against which it is directed.

20. Recombinant adenoviral vector according to Claim 19, **characterized in that** the said antibody is modified by fusion to a toxic or immunopotentiating substance.

21. Recombinant adenoviral vector according to one of Claims 15 to 18, derived from an adenovirus of human, canine, avian, bovine, murine, ovine, porcine or simian origin or from a hybrid comprising adenoviral genome fragments of different origins.

22. Recombinant adenoviral vector according to one of Claims 15 to 21, **characterized in that** it is defective for replication.

23. Recombinant adenoviral vector according to Claim 22, **characterized in that** it at least lacks all or part of the E1 region and, optionally, all or part of the E3 region.

24. Recombinant adenoviral vector according to Claim 22 or 23, comprising an exogenous nucleotide sequence encoding the heavy chain of the 2F5 antibody, an IRES element and the light chain of the 2F5 antibody; the said exogenous nucleotide sequence being placed under the control of the elements necessary for its expression.

25. Recombinant adenoviral vector according to Claim 22 or 23, comprising an exogenous nucleotide sequence encoding the signal sequence and the extracellular I and II domains of the CD4 protein operably fused to the constant γ3 region (hinge region-CH2 and CH3) of the heavy chain of the 2F5 antibody.

26. Recombinant adenoviral vector according to Claim 22 or 23, comprising an exogenous nucleotide sequence encoding the signal sequence and the extracellular I and II domains of the CD4 protein operably fused to the constant γ3 region (hinge region-CH2 and CH3) of the heavy chain of the 2F5 antibody and operably fused to the mature human angiogenin.

27. Recombinant adenoviral vector according to one of Claims 15 to 26, **characterized in that** the elements necessary for the expression comprise a promoter selected from the group consisting of the adenoviral early promoter E1A, the late promoter MLP (Major Late Promoter), the murine or human PGK (Phosphoglycerate kinase) promoter, the SV40 virus early promoter, the RSV (Rous Sarcoma virus) virus promoter, a promoter which is specifically active in tumour cells and finally a promoter which is specifically active in the infected cells.

28. Infectious viral particle comprising a recombinant adenoviral vector according to one of Claims 15 to 27.

29. Eukaryotic host cell comprising a recombinant adenoviral vector according to one of Claims 15 to 27 or an infectious viral particle according to Claim 28.

30. Pharmaceutical composition comprising a recombinant adenoviral vector according to one of Claims 15 to 27, an infectious viral particle according to Claim 28 or a eukaryotic host cell according to Claim 29, in association with a pharmaceutically acceptable carrier.

31. Pharmaceutical composition according to Claim 30, comprising 10⁴ to 10¹⁴ pfu.

32. Pharmaceutical composition according to Claim 30 or 31, **characterized in that** it is in injectable form.

33. Use of a recombinant adenoviral vector according to one of Claims 15 to 27, of an infectious viral particle according to Claim 28 or of a eukaryotic host cell according to Claim 29 for the preparation of a pharmaceutical composition intended for the treatment and/or prevention of the human or animal body by gene therapy.

34. Use according to Claim 33, for the preparation of a pharmaceutical composition intended for the treatment and/or prevention of acquired diseases and especially cancers and AIDS.

35. Use according to Claim 34, for the preparation of a pharmaceutical composition administrable via the intravenous or intratumour route.


### LISTE DE SEQUENCES

(1) INFORMATION GENERALE:
   (i) DEPOSANT:
      (A) NOM: Transgene S.A.
      (B) RUE: 11 rue de Molsheim
      (C) VILLE: Strasbourg
      (E) PAYS: France
      (F) CODE POSTAL: 67082
      (G) TELEPHONE: (33) 88 27 91 00
      (H) TELECOPIE: (33) 88 22 58 07
   (ii) TITRE DE L' INVENTION: Nouvel implant et nouveau vecteur pour le traitement des maladies acquises
   (iii) NOMBRE DE SEQUENCES: 20
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: Patent In Release #1.0, Version 01.25 (OEB)
(2) INFORMATION POUR LA SEQ ID NO: 1:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (B) SOUCHE: oligonucleotide de synthèse OTG5168
   (xi) DESCRIPTION DE LA SEQUENCE: SEQ ID NO: 1:
   GGAAGCTTCC ATGGACATGA GGGTC 25
(2) INFORMATION POUR LA SEQ ID NO: 2:
   (i) CARACTERISTIQUES DE LA SEQUENCE:
      (A) LONGUEUR: 25 paires dé bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (B) SOUCHE: oligonucleotide de synthèse OTG5169
   (xi) DESCRIPTION DE LA SEQUENCE: SEQ ID NO: 2:
   AAGAATTCCT AACACTCTCC CCTGT 25
(2) INFORMATION POUR LA SEQ ID NO: 3:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (B) SOUCHE: oligonucleotide de synthèse OTG5170
   (xi) DESCRIPTION DE LA SEQUENCE: SEQ ID NO: 3:
   AAAAGCTTCC ATGGAGTTGG GTCTG 25
(2) INFORMATION POUR LA SEQ ID NO: 4:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (B) SOUCHE: oligonucleotide de synthèse OTG5171
   (xi) DESCRIPTION DE LA SEQUENCE: SEQ ID NO: 4:
   GGGAATTCTC ATTTAGCCGG AGACA 25
(2) INFORMATION POUR LA SEQ ID NO: 5:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (B) SOUCHE: oligonucleotide de synthèse OTG6114
   (xi) DESCRIPTION DE LA SEQUENCE: SEQ ID NO: 5:
   GGGAATTCCA CCATGGGCAT CAAGATG 27
(2) INFORMATION POUR LA SEQ ID NO: 6:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (B) SOUCHE: oligonucleotide de synthèse OTG6115
   (xi) DESCRIPTION DE LA SEQUENCE: SEQ ID NO: 6:
   GGTCTAGATC TAACACTCAT TCCTGTTGAA 30
(2) INFORMATION POUR LA SEQ ID NO: 7:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (B) SOUCHE: oligonucleotide de synthèse OTG6192
   (xi) DESCRIPTION DE LA SEQUENCE: SEQ ID NO: 7:
   CTGTCGACCA CCATGGATGG AGCAGAG 27
(2) INFORMATION POUR LA SEQ ID NO: 8:
   (i) CARACTERISTIQUES DE LA SEQUENCE:
      (A) LONGUEUR: 43 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (B) SOUCHE: oligonucleotide de synthèse OTG6194
   (xi) DESCRIPTION DE LA SEQUENCE: SEQ ID NO: 8:
   ACGAATTCGC GGCCGCGCTC CCTCCGCCAC CTTTACCCGG AGT 43
(2) INFORMATION POUR LA SEQ ID NO: 9:
   (i) CARACTERISTIQUES DE LA SEQUENCE:
      (A) LONGUEUR: 26 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (8) SOUCHE: oligonucleotide de synthèse OTG5147
   (xi) DESCRIPTION DE LA SEQUENCE: SEQ ID NO: 9:
   CTGTGGCGGC CGCCGCACAG GTTATC 26
(2) INFORMATION POUR LA SEQ ID NO: 10:
   (i) CARACTERISTIQUES DE LA SEQUENCE:
      (A) LONGUEUR: 28 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (B) SOUCHE: oligonucleotide de synthèse OTG5148
   (xi) DESCRIPTION DE LA SEQUENCE: SEQ ID NO: 10:
   CAGGCGGCCG CTTTTTTCGT TATCTGAT 28
(2) INFORMATION POUR LA SEQ ID NO: 11:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (B) SOUCHE: oligonucleotide de synthèse OTG5299
   (xi) DESCRIPTION DE LA SEQUENCE: SEQ ID NO: 11:
   TACATTACAG CCTCAGAAGC A 21
(2) INFORMATION POUR LA SEQ ID NO: 12:
   (i) CARACTERISTIQUES DE LA SEQUENCE:
      (A) LONGUEUR: 23 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (B) SOUCHE: oligonucleotide de synthèse OTG6193
   (xi) DESCRIPTION DE LA SEQUENCE: SEQ ID NO: 12:
   ACGAATTCTC ATTTACCCGG AGT 23
(2) INFORMATION POUR LA SEQ ID NO: 13:
   (i) CARACTERISTIQUES DE LA SEQUENCE:
      (A) LONGUEUR: 35 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: ADNc CD4 humain
      (B) SOUCHE: oligonucleotide de synthese oTG7094
   (xi) DESCRIPTION DE LA SEQUENCE: SEQ ID NO: 13:
   CCGCTCGAGC CACCATGAAC CGGGGAGTCC CTTTT 35
(2) INFORMATION POUR LA SEQ ID NO: 14:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (A) ORGANISME: ADNc CD4 humain
      (B) SOUCHE: oligonucleotide de synthese oTG7095
   (xi) DESCRIPTION DE LA SEQUENCE: SEQ ID NO: 14:
   ACAAGATTTG GGCTCCTGGA AAGCTAGCAC 30
(2) INFORMATION POUR LA SEQ ID NO: 15:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: ADNc de la chaîne lourde de l'anticops 2F5
      (B) SOUCHE: oligonucleotide de synthese (oTG7097)
   (xi) DESCRIPTION DE LA SEQUENCE: SEQ ID NO: 15:
   GTGCTAGCTT TCCAGGAGCC CAAATCTTGT 30
(2) INFORMATION POUR LA SEQ ID NO: 16:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (A) ORGANISME: ADNc de la chaine lourde de l'anticorps 2F5
      (B) SOUCHE: oligonucleotide de synthèse (oTG7096)
   (xi) DESCRIPTION DE LA SEQUENCE: SEQ ID NO: 16:
   TGGGCCCGGG ATGGGGGCAG GGTGTACACC TGTGGT 36
(2) INFORMATION POUR LA SEQ ID NO: 17:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: ADNc de l'angiogenine humaine
      (B) SOUCHE: oligonucleotide de synthèse (OTG10089)
   (xi) DESCRIPTION DE LA SEQUENCE: SEQ ID NO: 17:
   GGGGGATCCC AGGATAACTC CAGGTAC 27
(2) INFORMATION POUR LA SEQ ID NO: 18:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (A) ORGANISME: ADNc de l'angiogenine humaine
      (B) SOUCHE: oligonucleotide de synthese (OTG10090)
   (xi) DESCRIPTION DE LA SEQUENCE: SEQ ID NO: 18:
   GGGGAATTCT TACGGACGAC GGAAAAT 27
(2) INFORMATION POUR LA SEQ ID NO: 19:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: ADNc de la chaine lourde de l'anticorps 2F5
      (B) SOUCHE: oligonucleotide de synthese (oTG10087)
   (xi) DESCRIPTION DE LA SEQUENCE: SEQ ID NO: 19:
   TGCCCCCATC CCGGGAGGAG ATGACCAAGA 30
(2) INFORMATION POUR LA SEQ ID NO: 20:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (A) ORGANISME: ADNc de la chaine lourde de l'anticorps 2F5
      (B) SOUCHE: oligonucleotide de synthèse (OTG10088)
   (xi) DESCRIPTION DE LA SEQUENCE: SEQ ID NO: 20:
   GGGGGATCCC CCGCCACCTT TAGCCGGAGA CAGGGA 36